(19) Europäisches Patentamt
European Patent Office
Office européen des brevets

(11) EP 2 359 138 B1

(12) **EUROPEAN PATENT SPECIFICATION**

(45) Date of publication and mention of the grant of the patent:
**04.05.2016 Bulletin 2016/18**

(21) Application number: **09830496.7**

(22) Date of filing: **04.12.2009**

(51) Int Cl.:
***G01N 33/543*** *(2006.01)* ***C07K 19/00*** *(2006.01)*
***G01N 33/547*** *(2006.01)*

(86) International application number:
**PCT/JP2009/070716**

(87) International publication number:
**WO 2010/064734 (10.06.2010 Gazette 2010/23)**

(54) **IMMOBILIZATION SUBSTRATE AND METHOD FOR PRODUCING THE SAME**

IMMOBILISIERUNGSSUBSTRAT UND VERFAHREN ZU SEINER HERSTELLUNG

SUBSTRAT D' IMMOBILISATION ET PROCÉDÉ POUR PRODUIRE CELUI-CI

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.12.2008 JP 2008309839**
**13.05.2009 JP 2009117081**

(43) Date of publication of application:
**24.08.2011 Bulletin 2011/34**

(73) Proprietors:
- **FUJIFILM Corporation**
**Tokyo 106-8620 (JP)**
- **The University of Tokyo**
**Bunkyo-ku,**
**Tokyo 113-8654 (JP)**

(72) Inventors:
- **MINAMI, Koichi**
**Ashigarakami-gun**
**Kanagawa 258-8577 (JP)**
- **TSUZUKI, Hirohiko**
**Ashigarakami-gun**
**Kanagawa 258-8577 (JP)**
- **UEDA, Hiroshi**
**Tokyo 113-8654 (JP)**
- **IHARA, Masaki**
**Nagano 399-4598 (JP)**

(74) Representative: **Hoffmann Eitle**
**Patent- und Rechtsanwälte PartmbB**
**Arabellastraße 30**
**81925 München (DE)**

(56) References cited:
**WO-A1-2010/047419 WO-A2-2005/067692**
**WO-A2-2008/048300 JP-A- 1 150 856**
**JP-A- 5 034 346 JP-A- 11 242 029**
**JP-A- 2003 075 443 JP-B2- 4 133 344**

- **UEDA ET AL: "OPEN SANDWICH ELISA: A NOVEL IMMUNOASSAY BASED ON THE INTERCHAIN INTERACTION OF ANTIBODY VARIABLE REGION", NATURE BIOTECHNOLOGY, NATURE PUBLISHING GROUP, NEW YORK, NY, US, vol. 14, 14 December 1996 (1996-12-14), pages 1714-1718, XP002085341, ISSN: 1087-0156, DOI: 10.1038/NBT1296-1714**
- **BALLERSTADT R ET AL.: 'Competitive-binding assay method based on fluorescence quenching of ligands held in close proximity by a multivalent receptor' ANAL CHIMACTA vol. 345, no. 1/3, 1997, pages 203 - 212, XP000904936**
- **WATANABE JUNJI ET AL.: 'Single step diagnosis system using the FRET phenomenon induced by antibody-immobilized phosphorylcholine group-covered polymer nanoparticles' SENS ACTUATORS B vol. 129, no. 1, 29 January 2008, pages 87 - 93, XP022424849**
- **AXEL JOHANNSSONA ET AL.: 'A fast highly sensitive colorimetric enzyme immunoassay system demonstrating benefits of enzyme amplification in clinical chemistry' CLIN CHIM ACTA vol. 148, no. 2, 1985, pages 119 - 124, XP023398930**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 359 138 B1

- **HIDEHITO SAITO ET AL.: 'Behavior of a cell surface receptor, RAGE: analysis by FRET using a monoclonal antibody' THE 80TH ANNUAL MEETING OF THE JAPANESE BIOCHEMICAL SOCIETY 2007, page 2P-0250, XP008148847**

## Description

### Technical Field

The invention relates to an immobilization substrate and a method for producing the immobilization substrate.

### Background Art

An immunoassay such as an ELISA is well known as a system which can precisely detect various target proteins. The immunoassay uses a protein and a specific antibody against this protein as a target substance and a detection substance, respectively, and detects the target substance with high sensitivity based on a specific interaction between these substances. The ELISA has undergone various improvements in consideration of sensitivity and operability. In recent years, an assay for detecting a large quantity of samples conveniently and with high sensitivity in a short time has been demanded.

A molecular imprinting technique is another system that effectively uses such a specific binding reaction between a target substance and a detection substance.

For example, Japanese Patent Application Laid-Open (JP-A) No. 2006-138656 discloses a molecular imprinting gel obtained by reacting a complex composed of a monomer or polymer introducing a ligand for a target molecule and the target molecule with a polymer cross linking agent to make a gel containing the complex composed of the ligand and the target molecule, and removing the target molecule from the gel. Furthermore, JP-ANo. 2006-138656 discloses that, when an antigen AFP is added to a gel substrate including an anti-AFP antibody and lectin, the gel is shrunk due to the binding of AFP to the anti-AFP antibody and lectin, and that the presence of AFP (target molecule) can be detected by such swelling and shrinking of the gel.

However, in the molecular imprinting method described above, since an immobilization substrate is formed by immobilizing an antigen-antibody complex by polymerization, the antigen to be detected must penetrate into a cross-linked gel matrix structure in order to reach the antibody. Thus it takes time for the antigen to react with the antibody, thereby requiring contacting a highly concentrated antigen with the substrate for two to four hours in order for a substance to be surely recognized. Furthermore, when the antigen is washed away and removed, efficient washing cannot be attained due to the same reason as above, whereby responsiveness after removal tends to be decreased. Therefore, rapid and highly sensitive detection cannot be achieved.

In addition to the above-described technique, as a system using a specific binding based on the antigen-antibody reaction, an immunoassay reagent that utilizes fluorescence resonance energy transfer between two types of fluoresce-labeled antibody fragments is disclosed in JP-A No.10-78436. In this immunoassay reagent, when an antigen is absent, an antigen-antibody complex is not formed and thus fluorescence resonance energy transfer (FRET) does not occur. On the other hand, when an antigen is present, an antigen-antibody complex is formed and thus FRET occurs. Therefore, the presence of an antigen can be detected rapidly and simply. However, in this reagent, since the antigen and the antibody fragment are not immobilized onto the substrate, the antigen and the antibody fragment may wash out or disperse due to the washing operation or the like. Therefore, the reagent is for one use only, and it is difficult to apply the reagent for repeated use. Even when these antibody fragments are simply immobilized on a substrate in order to use the reagent repeatedly, it is difficult to locate a luminescent substance and a luminescence recognizing substance in an adjacent position on the substrate. When the immobilization amount is increased in order to locate these substances in an adjacent position, a large quantity of luminescent substance and luminescence recognizing substance are adjacently located, which may result in an extremely poor S/N ratio.

In order to avoid the problem described above, JP-ANo. 2007-40834 discloses a method in which two antibody fragments are connected via a linker. In this method, while the antibody fragments can be immobilized in an adjacent position, the linker must be designed for every antibody fragment and antigen such that two antibodies can function effectively. Therefore, this method lacks versatility, thereby increasing the production costs.

As a simple detection system that can be repeatedly used, JP-A No. 2008-83042 discloses a reusable biosensor using a molecular recognition element and a nano particle. However, since the linker must be designed, this technique lacks versatility and requires much labor.

Further, as a simple detection system which can be repeatedly used, the Abstracts of the 57th Annual Meeting of the Japan Society for Analytical Chemistry (2008) p. 97 E3017 discloses a detection system using a substrate onto which an antibody labeled with a hydrophobic field-responsive fluorescent substance is immobilized. However, in this system, the hydrophobic field must be formed at an interface between an antigen and an antibody upon antigen recognition. Therefore, this system is limited only to a specific combination of antigen and antibody, and thus lacks versatility. In addition, the fluorescent substance needs to be labeled adjacent to the antigen recognition site of the antibody, which requires a highly sophisticated design. Therefore, this system requires high costs and much labor.

Ueda et al. disclose an immunoassay based on the interchain interaction of separated $V_L$ and $V_H$ chains from

a single chain antibody variable region (Ueda et al. (1996) Open sandwich ELISA: A novel immunoassay based on the interchain interaction of antibody variable region. Nature Biotechnology 14:1714-1718). In the presence of the antigen, the chains reassociate. $V_L$ fragments of anti-hen egg lysozyme (HEL) antibody HyHEL-10 were immobilized on microtiter plates. Samples were coincubated with an M13-displayed $V_H$ chain, and assayed with peroxidase-labeled anti-M13 antibody. Signal was detected in direct proportion to the amount of HEL in the sample.

In WO 2010/047419 an antibody-fragment-immobilizing substrate including a substrate and at least one set of antibody fragments, wherein antibody fragments of each set includes at least two types of separate antibody fragments that are capable of recognizing one type of antigen and that are independently immobilized on the substrate in a positional relationship that allows each of the antibody fragments in one set to bind to the same antigen is disclosed.

Summary of Invention

Any of the above-described techniques are not sufficient as a substrate for detecting a test substance that can precisely and simply detect a target molecule in a short time and that can be repeatedly used after washing.

In order to use such substrate for a biosensor, a manufacturing method with greater versatility has also been required, form the viewpoint of the production costs.

The present invention has been made in consideration of the above-described circumstances. The present invention provides an immobilization substrate that can precisely and simply detect a targeted molecule in a short time and that can be repeatedly used after washing.

The present invention also provides a method that can efficiently produce an immobilization substrate having greater versatility.

The present invention includes the following aspects:

<1> An antibody-fragment-immobilizing substrate comprising a substrate and at least one set of antibody fragments, wherein antibody fragments of each set include at least two types of separate antibody fragments,
the at least two types of separate antibody fragments include at least one type of labeled antibody fragment having a labeled site labeled with a luminescent substance and at least one type of acceptor antibody fragment having an acceptor site for accepting emission from the labeled antibody fragment,
the at least one type of labeled antibody fragment and the at least one type of acceptor antibody fragment are capable of cooperatively recognizing one type of antigen and are independently immobilized on the substrate in a positional relationship that allows each of the antibody fragments in one set to bind to the same antigen, wherein upon binding the antigen the at least one type of labeled antibody fragment and the at least one type of acceptor antibody fragment approach one another, whereby a reaction based on luminescence occurs between said antibody fragments, and
the antibody-fragment-immobilizing substrate is obtained by

contacting the separate antibody fragments with the antigen, and forming a complex whereby each of the antibody fragments binds to the antigen;
immobilizing the complex on a substrate via the antibody fragments in the complex; and
removing the antigen from the complex,
wherein the contacting includes mixing the antibody fragments and the antigen such that the ratio of the number of antigens to the valencies of molecules formed by a combination of antibody fragments is from 0.1:1 to 10:1.

<2> The antibody-fragment-immobilizing substrate according to <1>, wherein the at least two types of antibody fragments comprise a VH-region polypeptide and a VL-region polypeptide.

<3> The antibody-fragment-immobilizing substrate according to <1> or <2>, further comprising a polymer layer and wherein the at least two types of antibody fragments are immobilized on the polymer layer.

<4> The antibody-fragment-immobilizing substrate according to <3>, wherein the thickness of the polymer layer is from 1 nm to 0.5 mm.

<5> The antibody-fragment-immobilizing substrate according to <3> or <4>, wherein the polymer layer is bound to the substrate through a self-assembled monolayer.

<6> The antibody-fragment-immobilizing substrate according <5>, wherein the thickness of the self-assembled monolayer is from 0.2 nm to 10 μm.

<7> The antibody-fragment-immobilizing substrate according to any one of <3> to <6>, wherein the polymer is a polysaccharide.

<8> The antibody-fragment-immobilizing substrate according to any one of <1> to <7>, wherein the luminescent substance is at least one selected from a fluorescent dye or a fluorescent protein.

<9> The antibody-fragment-immobilizing substrate according to any one of <1> to <8>, wherein a fluorescence resonance energy transfer occurs between the labeled antibody fragment and the acceptor antibody fragment.

<10> Use of an antibody-fragment-immobilizing substrate as defined in any one of <1> to <9> for a bioreactor or biosensor based on a binding reaction between the at least two types of antibody fragments and the antigen.

<11> A method for producing an antibody-fragment-immobilizing substrate comprising:

contacting separate antibody fragments of at least two types that include at least one type of labeled antibody fragment having a labeled site labeled with a luminescent substance and at least one type of acceptor antibody fragment having an acceptor site for accepting emission from the labeled antibody fragment and that are capable of cooperatively recognizing one type of antigen, with the antigen, and forming a complex whereby each of the antibody fragments binds to the antigen;
immobilizing the complex on a substrate via the antibody fragments in the complex; and
removing the antigen from the complex to obtain the antibody-fragment-immobilizing substrate, wherein the antibody fragments are independently immobilized on the substrate in a positional relationship that allows each of the antibody fragments to bind to the same antigen
wherein the contacting includes mixing the antibody fragments and the antigen such that the ratio of the number of antigens to the valencies of molecules formed by a combination of antibody fragments is from 0.1:1 to 10:1.

<12> The method for producing the antibody-fragment-immobilizing substrate according to <11>, wherein the antibody fragments comprise a VH-region polypeptide and a VL-region polypeptide.

<13> The method for producing the antibody-fragment-immobilizing substrate according to <11> or <12>, wherein the removing is performed such that the avidity of the antibody fragments and the antigen in the complex is reduced.

According to the present invention, an antibody-fragment-immobilizing substrate that can precisely and simply detect a targeted molecule in a short time and that can be repeatedly used after washing is provided. The present invention also provides a method that can efficiently produce an antibody-fragment-immobilizing substrate having greater versatility.

Brief Description of Drawings

Figures 1A and 1B is a schematic diagram illustrating an example of the immobilization substrate according to the present invention. Figure 1A represents a schematic diagram of the immobilization substrate in the absence of the antigen.
Figure 1B represents a schematic diagram of the immobilization substrate in the presence of the antigen.
Figure 2 is a scheme for preparing the expression vectors according to Examples described in the present invention.

Modes for Carrying out the Invention

The immobilization substrate according to the present invention is an antibody-fragment-immobilizing substrate including a substrate and at least one set of antibody fragments, wherein antibody fragments of each set include at least two types of separate antibody fragments, the at least two types of separate antibody fragments include at least one type of labeled antibody fragment having a labeled site labeled with a luminescent substance and at least one type of acceptor antibody fragment having an acceptor site for accepting emission from the at least one type of labeled antibody fragment, and the at least one type of labeled antibody fragment and the at least one type of acceptor antibody fragment are capable of cooperatively recognizing one type of antigen and are independently immobilized on the substrate in a positional relationship that allows each of the antibody fragments in one set to bind to the same antigen.
In an embodiment, the immobilization substrate according to the present invention is an antibody-fragment-immobilizing substrate including a substrate and at least one set of antibody fragments, wherein the antibody fragments of each set include at least two different and separate antibody fragments that include at least one labeled antibody fragment having a labeled site labeled with a luminescent substance and at least one acceptor antibody fragment having an acceptor site for accepting emission from the at least one type of labeled antibody fragment, and the at least one labeled antibody fragment and the at least one acceptor antibody fragment are capable of cooperatively recognizing one type of antigen and are independently immobilized on the substrate in a positional relationship allowing each of the antibody fragments in one set to bind to the same antigen. For example, the present invention provides an immobilization substrate, on which at least two types of separate antibody fragments that include at least one type of labeled antibody fragment having a labeled site labeled with a luminescent substance and at least one type of acceptor antibody fragment having an acceptor site for accepting emission from the at least one type of labeled antibody fragment and that are capable of cooperatively recognizing one type of antigen are independently immobilized on a positional relationship that allows each of the antibody fragments to bind to the antigen.

More specifically, the antibody-fragment-immobilizing substrate may include, for example, a substrate and at least one (for example two or more) antibody-fragment set consisting of two or more different and separate antibody fragments that include at least one type of labeled antibody fragment having a labeled site labeled with a luminescent substance and at least one type of acceptor antibody fragment having an acceptor site for accepting emission from the at least one type of labeled antibody fragment and that are capable of recognizing one antigen, are independently immobilized on the substrate in a positional relationship whereby each of the two or more antibody fragments are capable of binding to the same antigen molecule or anything that forms a single antigen-bearing entity. Therefore, the antibody-fragment-immobilizing substrate of the present invention may include two or more such antibody-fragment sets that may bind to the same antigen-presenting entity or respectively different antigen-presenting entities.

Here, the "separate antibody fragments" represent antibody fragments that are not linked to each other (for example, by disulfide bonds).

In the antibody-fragment-immobilizing substrate according to the present invention, since at least two separate antibody fragments that are capable of recognizing one antigen are independently immobilized on the substrate in a positional relationship allowing the at least two separate antibody fragments in one set to bind the same antigen, the antibody fragments in one set are independent of one another and are placed on the substrate at positions adjacent to one another. Antibody fragments such as these that cooperatively recognize the antigen can exhibit a higher affinity than antibody fragments that are randomly immobilized. In addition, since the each of the antibody fragments are independently immobilized on the substrate in a positional relationship allowing the at least two separate antibody fragments in one set bind to the same antigen, and since each antibody fragment is bound to the substrate through a part of the antibody, a moiety having the antigen recognition site is allowed to move such a degree that it can bind to the antigen. Therefore, when the antigen exists, each antibody fragment in one set can easily approach the antigen and can cooperatively bind to the antigen in a short time. Since dispersion or washing away of the respective antibody fragments during washing can be suppressed, the substrate can be used for repeated measurement. Further, since antibody fragments in one set form a complex with an antigen and are immobilized to the substrate as a complex, antibody fragments constituting different complexes may be placed on the substrate at positions nonadjacent to one another. Therefore, even in an immobilization substrate for measuring multiple test substances simultaneously, the antibody fragments in one set are at positions on the substrate nonadjacent to other sets of antibody fragments which recognize a different antigen, thereby cross talk between antibody fragments, which is usually problematic in ELISA, can be suppressed.

Furthermore, on the immobilization substrate according to the present invention, at least two types of antibody fragments are immobilized, the at least two types of antibody fragments include at least one type of labeled antibody fragment having an labeled site labeled with the luminescent substance and at least one acceptor antibody fragment having an acceptor site accepting emission from the at least one type of labeled antibody fragment, and the at least one type of labeled antibody fragment and the at least one acceptor antibody fragment are capable of cooperatively recognizing one type of antigen. Therefore, when the antigen exists, the at least one type of labeled antibody fragment and the at least one type of acceptor antibody fragment approach one another, whereby a reaction based on luminescence, such as FRET or BRET, easily occurs between these antibody fragments. Based on such a reaction, precise and simple detection of antigens can be performed in a short time.

Although prior documents disclose two types of antibodies that are connected by a linker, the distance between these antibodies varies depending on a combination of the antigen and antibody to be used, and thus a suitable linker depending on the type of the antigen must be designed. In addition, regarding the labeling with a luminescent substance, the luminescent substance and an emission-accepting substance need to be selectively labeled with the same molecule, thereby requiring very high costs and a large amount of labor for production. On the other hand, the present invention does not require connecting the two types of the antibody fragments with a linker, and thus can be applied for any antigen-antibody combination. In addition, in the present invention, antibodies can be labeled independently. Therefore, the method according to the present invention has greater versatility and is particularly suitable for production.

Hereinafter, the present invention is further described.

(I) Immobilization substrate

(1) Substrate

Preferable examples of the substrate of the invention include a substrate in which a functional group is added to any of the following materials: metal oxides such as an optical glass (for example, BK7), silica, alumina, titania, zirconia and indium tin oxide (ITO); metal nitrides such as silicon nitride, gallium nitride, aluminum nitride and indium nitride; and synthetic resins, specifically a resin such as Sepharose (tradename), polyethylene, polystyrene, poly (meth)acrylic acid, poly (meth)acrylamide, poly methyl (meth)acrylate, polyethylene terephthalate, polycarbonate or a cyclo-olefin polymer. Among these, materials transparent against a light source, such as glass, ITO, polymethyl (meth)acrylate, polyethylene terephthalate, polycarbonate or a cyclo-olefin polymer are preferable. Examples of the functional group include an amino group, a carboxy group, a maleimide group, an aldehyde group, a succinimide group, a thiol group, a hydrazine group, an isocyanate group, an epoxy group, a vinyl sulfone group, a vinyl group, and a cyano group.

The substrate is preferably a material that is not anisotropic under polarized light and has excellent workability.

Examples of a method for adding these functional groups include a known method of treating a surface such as plasma treatment, ozone treatment, etching treatment using an acid and/or alkali, or a method using a self-assembled monolayer. From the viewpoint of production suitability, a method using the self-assembled monolayer is preferable.

(2) Polymer layer

A polymer layer may be formed on the substrate of the present invention. Examples of a polymer constituting the polymer layer include a hydrophilic polymer, a hydrophobic polymer and a combination thereof. The polymer layer is preferably a layer consisting essentially of a hydrophilic polymer. The polymer layer may be directly linked to the substrate or may be indirectly linked to the substrate. Examples of a method of direct linking of the polymer layer to the substrate include a method in which a polymer is linked to a substrate via graft polymerization on the substrate. Examples of a method of indirect linking of the polymer layer include a method in which a hydrophobic polymer is applied onto a substrate, and then a hydrophilic polymer is linked to the hydrophobic polymer, and a method in which a compound capable of binding to the substrate and a polymer (hereinafter, sometimes referred to as a linker) is provided on the surface of the substrate, and then the polymer is linked to the compound. In one preferable embodiment, a self-assembled monolayer is used as a linker, and a hydrophilic polymer is linked to a glass substrate via the self-assembled monolayer. Hereinafter, this embodiment is described in detail. Examples of a method of forming the self-assembled monolayer include (2-1) a method using a silane coupling agent and (2-2) a method using alkanethiol. Each method will be described below.

(2-1) The method using a silane coupling agent

In the method using the silane coupling agent, by providing a silane coupling agent described below to the substrate described above, the self-assembled monolayer can be formed by the silane coupling agent, whereby the functional groups are provided on the substrate.

As the silane coupling agent that can be used in the present invention, a silicon-containing compound represented by the following Formula A-1 may be used to form a covalent bond such as substrate=oxygen-silicon-carbon, thereby providing the functional groups to the substrate surface. In Formula A-1, $X^a$ represents a functional group; $L^a$ represents a linker moiety such as a linear, branched or cyclic carbon chain; $R^a$ represents hydrogen or an alkyl group having 1 to 6 carbon atoms; $Y^a$ represents a hydrolyzable group; *m* and *n* each independently represent an integer of from 0 to 3, and the total of *m* and *n* is 3.

$$X^a\text{-}L^a\text{-}Si\text{-}(R^a{}_m)Y^a{}_n \qquad \text{Formula A-1}$$

Examples of the hydrolyzable group ($Y^a$) include an alkoxy group, a halogen group and an acyloxy group. More specifically, examples of the hydrolyzable group ($Y^a$) include a methoxy group, an ethoxy group and chlorine.

Specific examples of the silane coupling agent include γ-aminopropyl trimethoxysilane, N-β-(aminoethyl)-γ-aminopropyl trimethoxysilane, γ-aminopropyl methyldiethoxysilane, γ-mercaptopropyl trimethoxysilane, and γ-glycidoxypropyltriethoxysilane. Examples of the reaction method of the silane coupling agent include a general method such as

a method described in "Effects and usages of silane coupling agents" (Science & Technology Co., Ltd.).

Examples of the functional group ($X^a$) of the silane coupling agent are not limited as long as the functional group ($X^a$) can bind to the below-described polymer and/or complex. Examples thereof include functional groups such as an amino group, a carboxyl group, a hydroxyl group, an aldehyde group, a thiol group, an isocyanate group, an isothiocyanate group, an epoxy group, a cyano group, a hydrazino group, a hydrazide group, a vinyl sulfone group, a vinyl group and a maleimide group. A combination of these functional groups or a derivative thereof may also be used. Among these, functional group ($X^a$) is preferably an amino group or an epoxy group.

(2-2) The method using alkanethiol

In the method using alkanethiol, a metal film is disposed on a surface of the substrate described above, and then the alkanethiol is provided thereon. Here, "disposed on a surface of the substrate" means a case whereby the metal film is disposed on a surface of the substrate such that it directly comes into contact with the substrate, as well as a case whereby the metal film is disposed via another layer without directly coming into contact with a surface of the substrate.

These metals can be used singly or in combination of two or more kinds thereof. Moreover, in consideration of adherability to the substrate described above, an intermediate layer including chrome or the like may be provided between the substrate and the metal film.

The film thickness of the metal film is not particularly limited, and preferably from 0.1 nm to 1 mm, and more preferably from 1 nm to 1 μm. When the film thickness exceeds 1 mm, production cost increases. Moreover, when the intermediate layer including chrome or the like is provided, the thickness of the intermediate layer is preferably from 0.1 nm to 10 nm.

A method of coating a metal film using alkanethiol has been intensively studied by Professor Whitesides at Harvard University, and the details thereof are described in Chemical Review, 105,1103-1169 (2005), for example. When gold is used as the metal, an alkanethiol represented by the following Formula A-2 (wherein *n* represents an integer of from 3 to 20, and $X^b$ represents a functional group) may be used as an organic layer-forming compound, whereby a monomolecular film having an orientation can be formed in a self-assemble manner based on an Au-S bond and the van der Waals force between alkyl chains. A self-assembled monolayer may be produced by an extremely easy method, which includes inunersion of a gold substrate in a solution of an alkanethiol derivative. More specially, functional groups can be provided to a surface of the substrate, for example, by forming a self-assembled monolayer using a compound in which $X^b$ in Fonnula A-2 represents an amino group, a carboxyl group, a hydroxyl group, an aldehyde group, a thiol group, an isocyanate group, an isothiocyanate group, an epoxy group, a cyano group, a hydrazino group, a hydrazide group, a vinyl sulfone group, a vinyl group or a maleimide group.

$$HS(CH_2)_nX^b \qquad \text{A-2}$$

In Formula A-2, the number of repetition of an alkylene group, *n*, is preferably an integer of from 3 to 16, and more preferably an integer of from 4 to 8. The alkylene moiety may be substituted with a multiple bond or a hetero atom such as nitrogen or oxygen. When the alkyl chain of the alkanethiol derivative is too short, formation of the self-assembled monolayer is difficult. When the alkyl chain of the alkanethiol derivative is too long, water solubility is decreased and thus handling thereof is difficult.

A self-assembled monolayer can be formed with a single kind of alkanethiol of Formula A-2 (that is, an alkanethiol having one type of the functional group $X^b$). A self-assembled monolayer can also be formed from a mixture of two or more kinds of alkanethiols.

The film thickness of the self-assembled monolayer is not particularly limited, and is preferably from 0.2 nm to 10 μm, more preferably from 1 nm to 500 nm, and still more preferably from 5 nm to 50 nm. When the film thickness is 10 μm or less, a test substance can easily diffuse into the monolayer. When the film thickness is 0.2 nm or more, the immobilization amount of a substance to be immobilized on the substrate can be increased.

In the present invention, the antibody fragments may be directly immobilized to the self-assembled monolayer. However, in order to improve the antigen-binding efficiency of the antibody fragments, it is preferable to form a polymer layer on the self assembled monolayer so as to provide functional groups for immobilizing the antibody fragments on the substrate surface. The polymer used in the invention is preferably a hydrophilic polymer, and specific examples thereof include gelatin, agarose, chitosan, dextran, carrageenan, alginic acid, starch, cellulose, or derivatives thereof such as carboxymethyl derivative, and water-swellable organic polymers (for example, polyvinyl alcohol, polyacrylic

acid, polyacrylamide, polyethylene glycol and derivatives thereof.).

Preferable examples of the hydrophilic polymer that can be used in the invention include a carboxyl group-containing synthetic polymer and a carboxyl group-containing polysaccharide. Examples of the carboxyl group-containing synthetic polymer include polyacrylic acid, polymethacrylic acid and copolymers thereof. Further examples include a methacrylic acid copolymer, an acrylic acid copolymer, an itaconic acid copolymer, a crotonic acid copolymer, a maleic acid copolymer, a partially esterified maleic acid copolymer and a hydroxyl group-containing polymer in which an acid anhydride has been added thereto, such as those disclosed, for example, in JP-A No. 59-53836, from line 2, upper right column on page 3, to line 9, lower left column on page 6, and JP-A No. 59-71048, from line 1, lower left column on page 3, to line 3, upper left column on page 5.

Examples of the carboxyl group-containing polysaccharide include any one selected from extracts from natural plants, products obtained by fermentation by microorganisms, synthetic products obtained by enzymes and chemically synthetic products. Specific examples thereof include hyaluronic acid, chondroitin sulfate, heparin, dermatan sulfate, carboxymethyl cellulose, carboxyethyl cellulose, cellouronic acid, carboxymethyl chitin, (carboxymethyl dextran, and carboxymethyl starch. As a carboxyl group-containing polysaccharide, a commercially available product can also be used. Specific examples thereof include carboxymethyl dextrans such as CMD, CMD-L and CMD-D40 (trade names, all manufactured by Meito Sangyo Co., Ltd.), sodium carboxymethylcellulose (manufactured by Wako Pure Chemical Industries, Ltd.) and sodium alginate (manufactured by Wako Pure Chemical Industries, Ltd.).

The molecular weight of the hydrophilic polymer used in the invention is not particularly limited. In general, the weight average molecular weight is preferably from $2 \times 10^2$ to $5 \times 106$, and more preferably from $1 \times 10^4$ to $2 \times 10^6$. When the weight average molecular weight is less than the above range, the immobilization amount of the antibody fragments on the substrate may be reduced. A weight average molecular weight greater than the above range may result in high solution viscosity, thereby making the handling thereof difficult.

The thickness of the polymer layer in an aqueous solution is preferably from 1 nm to 0.5 mm, more preferably from 1 nm to 1 $\mu$m, and still more preferably from 100 nm to 500 nm. When the thickness is less than the above range, the immobilized amount of a physiologically active substance reduces, which makes interaction of the physiologically active substance with a test substance less likely to occur. When the film thickness exceeds the above range, the uniformity of the polymer layer may not be maintained, and a test substance may be inhibited from diffusing into the polymer film. In particular, when the interaction is detected from the side of a sensor substrate opposite to a hydrophilic polymer-immobilized surface thereof, the detection sensitivity may be decreased due to the long distance from the detection surface to an interaction-forming part.

The thickness of the hydrophilic polymer in an aqueous solution can be evaluated by, for example, AFM or ellipsometry.

When the carboxyl group-containing polymer is used, the complex can be immobilized on the substrate via functional groups provided to the substrate surface by activating carboxyl groups of the polymer. Examples of the method for activating the carboxyl group-containing polymer include a known method, for example, a method in which a carboxyl group-containing polymer is activated by using a water-soluble carbodiimide such as 1-(3-dimethylaminopropyl)-3-ethylcarbodiimide (EDC) and N-hydroxysuccinimide (NHS), and a method in which a carboxyl group-containing polymer is activated by using EDC alone. The polymer having the carboxyl group activated by using these methods can be reacted with the substance having an amino group so as to attach the hydrophilic polymer to the substrate.

Examples of the method for activating the carboxyl group-containing polymer also include a method using a nitrogen-containing compound. Specific examples of the nitrogen-containing compound include a compound represented by the following Formula (Ia) or Formula (Ib) (wherein, $R^1$ and $R^2$ each independently represent an unsubstituted or substituted carbonyl group, a carbon atom which may have a substituent, or a nitrogen atom which may have a substituent; $R^1$ and $R^2$ may be linked to each other to form a 5- or 6-membered ring; A represents a carbon atom having a substituent, or a phosphorus atom having a substituent; M represents an element which forms an (n-1)-valent counter ion as a whole; and X represents a halogen atom).

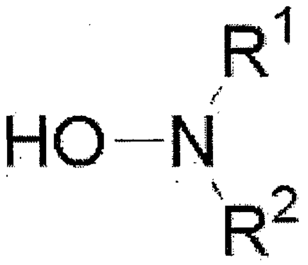

or

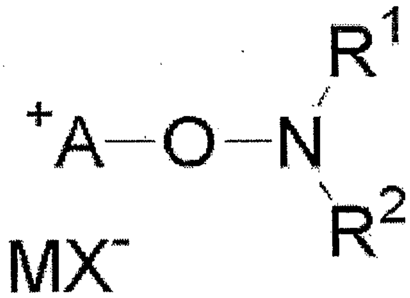

(Ia) (Ib)

In Formulae (Ia) and (Ib), $R^1$ and $R^2$ each independently represent an unsubstituted or substituted carbonyl group, a carbon atom which may have a substituent, or a nitrogen atom which may have a substituent, and preferably $R^1$ and $R^2$ are linked to each other to form a 5- or 6-membered ring. Preferable examples of the nitrogen-containing compound include hydroxysuccinimide, hydroxyphthalimide, 1-hydroxybenzotriazole, 3,4-dihydroxy-3-hydroxy-4-oxo-1,2,3-benzotriazine, and derivatives thereof.

Preferable examples of the nitrogen-containing compound also include the following compounds (Ic), (Id) and (Ie).

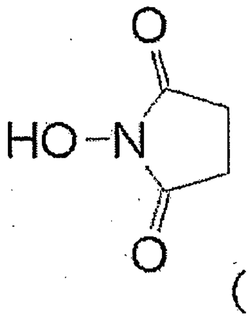

(Ic)

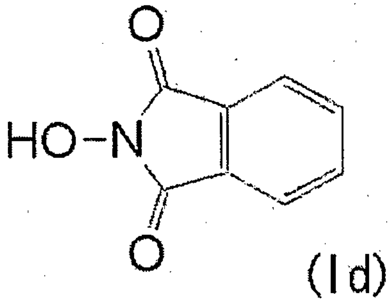

(Id)

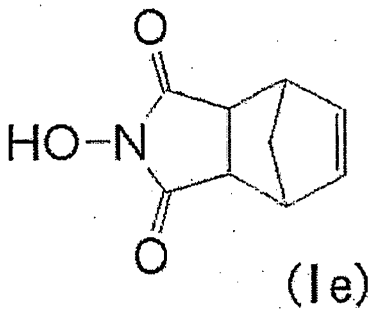

(Ie)

Preferable examples of the nitrogen-containing compound include a compound represented by the following Formula (II) (wherein, Y and Z each independently represent CH or a nitrogen atom).

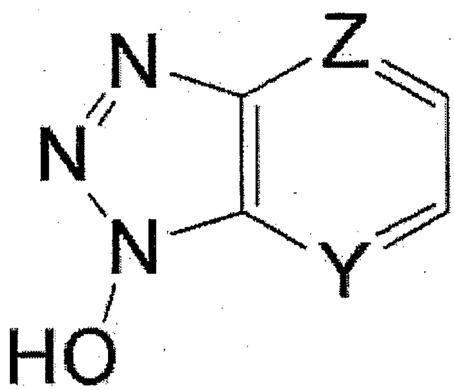

(II)

Preferable examples of the compound represented by Formula (II) include the following compounds (II-1) to (II-3).

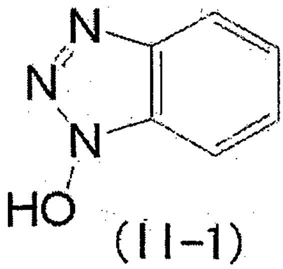
(II-1)

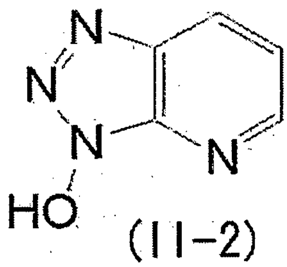
(II-2)

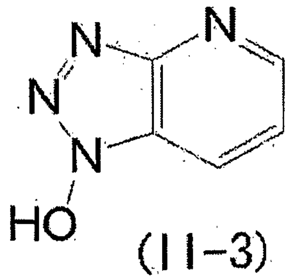
(II-3)

Preferable examples of the nitrogen-containing compound also include a compound represented by the following Formula (II-4).

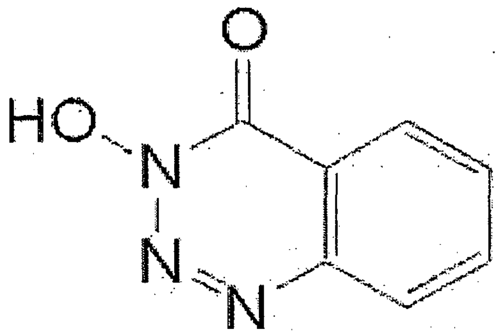
(II-4)

Preferable examples of the nitrogen-containing compound further include a compound represented by the following Formula (III) (wherein A represents a carbon atom having a substituent, or a phosphorus atom having a substituent; Y and Z each independently represent CH or a nitrogen atom; M represents an element which forms an (n-1)-valent counter ion as a whole; and X represents a halogen atom).

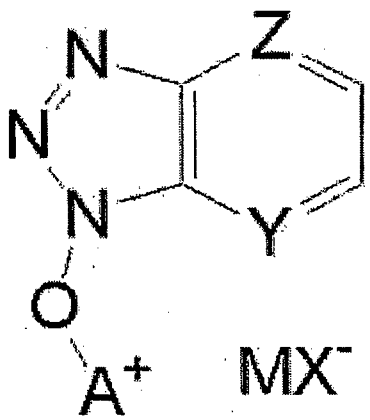
(III)

In Formula (III), the substituent which the carbon atom or phosphorus atom represented by A has is preferably an amino group having a substituent. More specifically, a dialkylamino group such as a dimethylamino group or a pyrrolidino group is preferable. Examples of the element represented by M include a phosphorus atom, a boron atom and an arsenic atom. Among these, a phosphorus atom is preferable. Examples of the halogen atom represented by X include a fluorine atom, a chlorine atom, a bromine atom and an iodine atom. Among these, a fluorine atom is preferable.

Specific examples of the nitrogen-containing compound represented by Formula (III) include the following compounds (III-1) to (III-6).

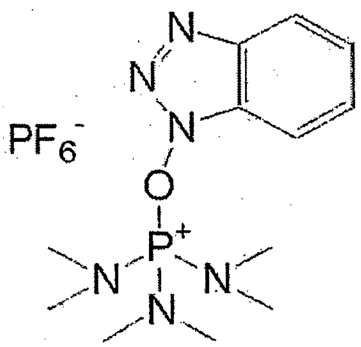

(III-1)

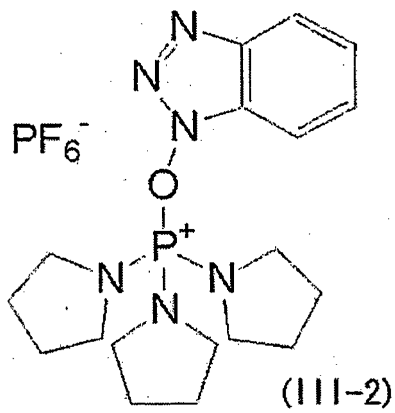

(III-2)

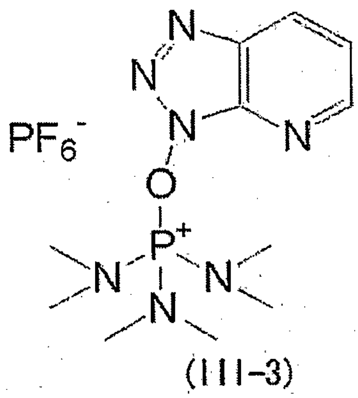

(III-3)

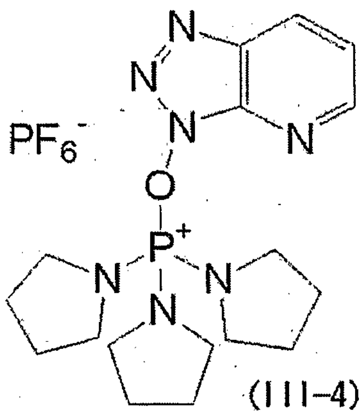

(III-4)

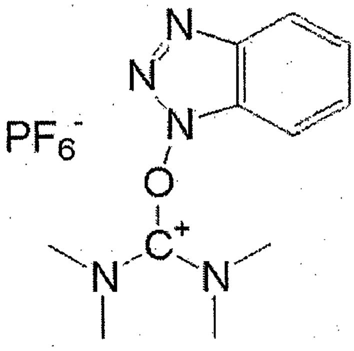

(III-5)

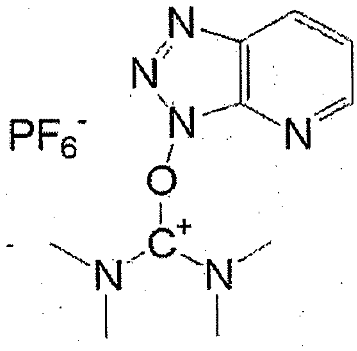

(III-6)

Preferable examples of the nitrogen-containing compound further include a compound represented by the following Formula (IV) (wherein A represents a carbon atom having a substituent, or a phosphorus atom having a substituent; M represents an element which forms an (n-1)-valent counter ion as a whole; and X represents a halogen atom).

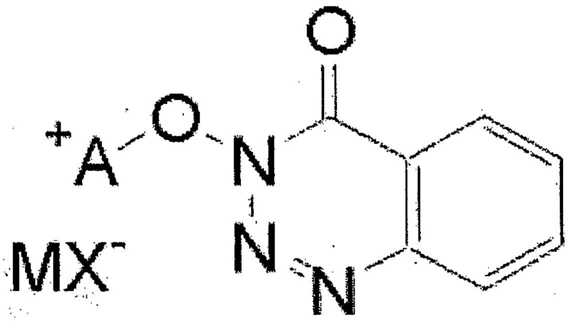

(IV)

Preferable examples of the nitrogen-containing compound represented by Formula (IV) include the following compound (IV-1).

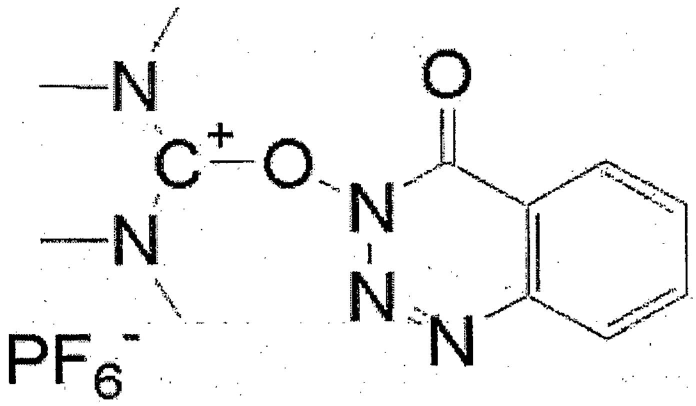

(IV-1)

Examples of the method for activating the carboxyl group-containing polymer also include a method using a phenol derivative having an electron-withdrawing group. The σ value of the electron-withdrawing group is preferably 0.3 or more. Specific examples of the phenol derivative having an electron-withdrawing group include the following compounds (V-1) to (V-4).

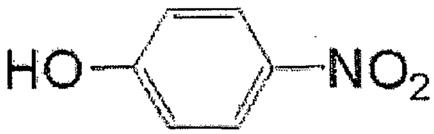

(V-1)

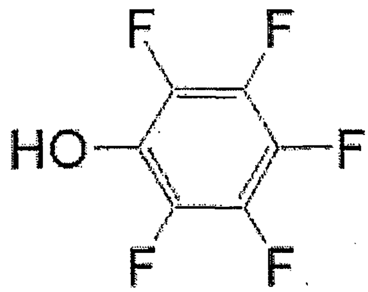

(V-2)

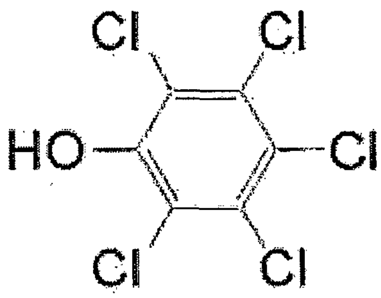

(V-3)

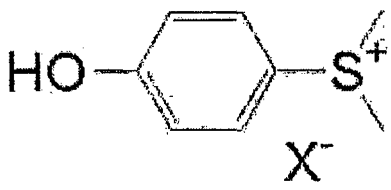

(V-4)

Any of the above-described carbodiimide derivative, nitrogen-containing compound and phenol derivative may be used singly, or in combination of two or more kinds thereof, if desired. It is preferable to use the carbodiimide derivative and the nitrogen-containing compound in combination.

Examples of the method for activating the carboxyl group-containing polymer further include a method using the following compound (V-6). The compound (V-6) may be used singly, or in combination with the carbodiimide derivative, the nitrogen-containing compound and/or the phenol derivative.

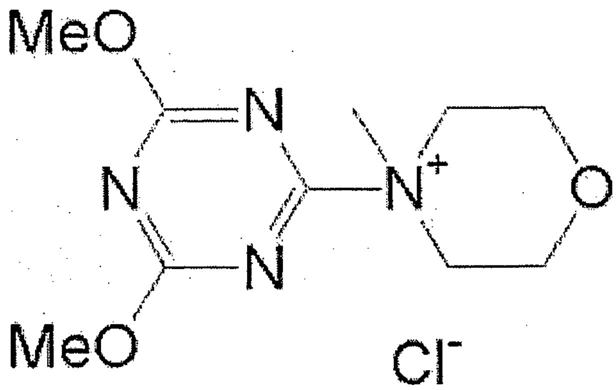

(V-6)

In addition, preferable examples of the method for activating the carboxyl group of the carboxyl group-containing polymer include methods such as that described in paragraph Nos. [0011] to [0022] of JP-A No.2006-58071 (i.e., a method of forming a carboxylic acid amide group by activating the carboxyl group on a surface of a substrate with a compound selected from a uronium salt having a particular structure, a phosphonium salt having a particular structure or a triazine derivative having a particular structure); and methods such as that described in paragraph Nos. [0011] to [0019] of JP-A No.2006-90781 (i.e., a method of forming a carboxylic acid amide group by activating the carboxyl group on a surface of a substrate with a carbodiimide derivative or a salt thereof, converting the activated group to an ester group using a nitrogen-containing heteroaromatic compound having a hydroxyl group, a phenol derivative having an electron-withdrawing group or an aromatic compound having a thiol group, and then reacting the resulting ester group with an amine).

The polymer containing an activated carboxyl group may be prepared as a polymer solution and reacted with the substrate. Alternatively, the polymer containing an activated carboxyl group of the present invention may be formed in a thin film on the substrate by using a method such as a spin coating method, and reacted with the substrate in the thin film state. The polymer is preferably reacted in the thin film state with the substrate.

As described above, the polymer of the invention is preferably reacted in the thin film state with the substrate. As a method for forming a thin film on the substrate, known methods can be used. Specific examples of such methods that can be used include an extrusion coating method, a curtain coating method, a casting method, a screen printing method, a spin coating method, a spray coating method, a slide bead coating method, a slit and spin method, a slit coating method, a die coating method, a dip coating method, a knife coating method, a blade coating method, a flow coating method, a roll coating method, a wire-bar coating method, and a transfer printing method. These methods are described in, for example, "Progress in Coating Technology (Coating Gijutsu no Shinpo)" written byYuji Harazaki, Sogo Gijyutsu Center (1988); "Coating Technology (Coating Gijutsu)" Technical Information Institute Co., Ltd. (1999); "Aqueous Coating Technology (Suisei Coating no Gijutsu)" CMC (2001); "Evolving Organic Thin Film: Edition for Deposition (Shinkasuru Yuuki Hakumaku: Seimaku hen)" Sumibe Techno Research Co., Ltd. (2004); and "Polymer Surface Processing Technology (Polymer Hyomen Kako Gaku)" written by Akira Iwamori, Gihodo Shuppan Co., Ltd. (2005). In the present invention, the method for forming a thin film on the substrate is preferably a spray coating method or a spin coating method, and more preferably a spin coating method, since a coating film having a controlled film thickness can be readily produced by such a method.

(2) Antibody fragment

In the present invention, at least one type of labeled antibody fragment having a labeled site labeled with a luminescent substance and at least one type of acceptor antibody fragment having an acceptor site for accepting emission from the labeled antibody fragment in one set cooperatively recognize one type of antigen

The antibody-fragment immobilization substrate of the present invention is not particularly limited as long as at least one antibody-fragment set containing the labeled and acceptor antibody fragments capable of recognizing one type of antigen is immobilized thereon. Plural types of antibody-fragment sets each consisting of such antibody fragments may be immobilized on the substrate. By using the plural types of antibody-fragment sets, plural types of antigens can be recognized with one immobilization substrate.

The type of the antigen is not specifically restricted as long as it can interact with an antibody, and can be appropriately selected depending on the target substance to be detected. Further, the antibody fragment can be appropriately selected as an antibody capable of interacting with such an antigen.

Any antibodies can be used as long as antibody fragments of two or more types can individually or cooperatively recognize and bind to one antigen. Examples of such plural antibody fragments include two or more types of antibody

fragments each having at least a part of an antigen recognition site for (the same) one epitope on one type of antigen, two or more types of antibody fragments each having an antigen recognition site for a different epitope on one type of antigen, and antibody fragments each having any one of plural hypervariable regions for one epitope. Such plural antibody fragments can also be used in combination, as long as the antibodies recognize one antigen.

Preferable examples of the antibody fragment of the invention include a VH-region polypeptide and VL-region-polypeptide, from the viewpoint of an antigen binding ability. The antibody fragment of the invention is more preferably a combination of a VH-region polypeptide and VL-region polypeptide, from the viewpoint of an antigen binding ability and versatility. The combination is not specifically limited, as long as one of a VH-region polypeptide or a VL-region polypeptide is used as a labeled antibody fragment and the other one is used as an acceptor antibody fragment.

The length of VH-region polypeptide and VL-region polypeptide may be either longer or shorter than the VH region and VL region of the antibody, respectively, as long as they can bind to a target antigen in association with each other. These polypeptides can be produced from a monoclonal antibody made by a hybridoma technique by using a conventional method. For example, the polypeptides can be obtained as follows.

First, a monoclonal antibody capable of recognizing a desired test substance is produced by a known method. The gene encoding the variable region of this antibody is then specified by a method using a cDNA library and hybridization technique, followed by cloning this gene into a vector. The sequence encoding the VH and/or VL region is then obtained from this recombinant vector, and this sequence fragment is subcloned into an expression vector. By expressing this gene in host cells, the required amount of VH and/or VL can be obtained.

In order to obtain the VH/VL coding sequence from the antibody gene, the desired sequence region may be isolated by cleavage with a restriction enzyme and amplified it using a cloning vector, or the desired sequence may be amplified by PCR. When VH and/or VL are/is expressed in host cells, a gene encoding any reporter molecule may also be cloned into the expression vector and VH and/or VL can be expressed as a fusion protein or a chimera protein with the reporter molecule.

In addition to the above methods, VH and/or VL can be obtained by proteolysis of the antibody molecule using a protease. This method has an advantage of saving time and effort on the gene cloning.

The VH-region polypeptide and the VL-region polypeptide may be a fusion product with a biomolecule. Such a fusion product has an advantage of improving the stability.

The biomolecule which can be fused with the VH-region polypeptide or the VL-region polypeptide is not particularly restricted, and examples thereof include alkaline phosphatase, protein G, eGFP, eYFP, β-galactosidase, GST, chitin binding protein (CBP), NusA, thioredoxin, DsbA, DsbC, and maltose binding protein (MBP). Among these, in order to further increase stability, MBP can be preferably used.

These fusion products can be produced by a conventional method. For example, the fusion product can be obtained by incorporating the gene encoding the biomolecule into a vector at the time of the above-described gene cloning so as to be expressed simultaneously, or by adding a linker to the VH-region polypeptide or the VL-region polypeptide to link with the biomolecule. The method for producing the fusion product can be appropriately selected depending on the type and size of the biomolecule to be fused.

An antibody fragment other than the VL-region polypeptide and the VH-region polypeptide can be produced in a manner similar to the method for producing the VL-region polypeptide and the VH-region polypeptide. Such a method is easily applicable by those skilled in the art.

The at least two types of antibody fragments immobilized on the substrate constitutes a set (combination) that is composed of at least one type of labeled antibody fragment and at least one type of acceptor antibody fragment and that is capable of recognizing one type of antigen. As long as the antibody fragments immobilized onto the substrate constitute such a set, the set may include not only two types but also three or more types of antibody fragments. When plural types of antibody fragments are used, the labeled antibody fragment(s) and acceptor antibody fragment(s) in one set can approach one another around one antigen. Consequently, the acceptor site of the acceptor antibody fragment can easily accept luminescence from the luminescent substance on the labeled antibody fragment. In consideration of emission accepting efficiency, it is preferable that labeled antibody fragments labeled with one type of luminescent substance and acceptor antibody fragments labeled with one type of emission-accepting substance are included in one set.

In the present invention, "the luminescent substance" broadly includes a substance capable of emitting light during a transition from an excited state caused by excitation light, oxidization reactions or the like, to a ground state. Further, "accept luminescence" used herein broadly includes the emitting of light or the occurrence of a chemical reaction caused directly or indirectly by excitation of the luminescent substance in an excited state. Both the labeled antibody fragment and the acceptor antibody fragment in the present invention may respectively be labeled with a luminescent substance, or only the labeled antibody may be labeled with a luminescent substance. When both of the labeled antibody fragment and acceptor antibody fragment are respectively labeled with a luminescent substance, each antibody fragment may be labeled with the luminescent substance at any site as long as binding of the antibody fragment with the antigen or the substrate is not inhibited. Examples of the label that can be introduced into the acceptor antibody fragment capable

of accepting luminescence of the luminescent substance include luminescent substances, but are not particularly restricted as long as the label can be repeatedly used. For example, the ALPHASCREEN kit (trade name, manufactured by PerkinElmer Co., Ltd.), which generates singlet oxygen and emits light from a fluorescent substance, can be used for labeling.

A method for labeling the antibody fragment with a luminescent substance can be appropriately selected depending on the type of the luminescent substance. When the luminescent substance is a nonpeptidic compound, the labeling can be carried out by a known method including a method in which a thiol group or an amino group of the antibody is chemically modified with a functional group such as a maleimide group or a succinimide group. When the luminescent substance is a peptidic compound such as a fluorescent protein, the labeled antibody fragment can be produced as a fusion protein with antibody fragment. Any known method can be used for producing the fusion protein.

The antibody fragment of the invention may be labeled with a luminescent substance at any position as long as the acceptor antibody fragment can accept luminescence when the labeled antibody fragment and the acceptor antibody fragment are bound to the antigen by an antigen-antibody reaction. The luminescent substance may be linked directly to the antibody fragment or may be linked via a spacer. The position of the luminescent substance may be appropriately adjusted by using the spacer such that the luminescence can be accepted when the antibody fragment labeled with the luminescent substance and the acceptor antibody fragment are bound to the antigen by the antigen-antibody reaction. Examples of the spacer include flexible hydrophilic molecules such as polyethylene glycol derivatives and peptides. In consideration of emission accepting efficiency, polyethylene glycol is preferable.

In consideration of accuracy and ease of detection or the like, preferable examples of the luminescent substances used for labeling the labeled antibody fragment or the acceptor antibody fragment include a fluorescent donor substance and acceptor substance, in which a fluorescence resonance energy transfer (FRET) or a bioluminescence resonance energy transfer (BRET) occurs between the luminescent substance on the labeled antibody fragment and the luminescent substance on the acceptor antibody fragment. It is preferable that each of the labeled antibody fragment and the acceptor antibody fragment is labeled with a luminescent substance (that is, fluorescent substance) between which FRET can occur.

The fluorescence resonance energy transfer described above is well known, as described in paragraphs [0158] to [0161] of examined Japanese patent publication (JP-B) No. 2007-523754, and in paragraph [0025] of JP-B No. 2001-519525. A variety of donors and acceptors used for fluorescence resonance energy transfer system are commercially available. Any combination of donors and acceptors can be used as long as the fluorescence resonance energy transfer occurs between the donor and acceptor, and can be appropriately selected based on the information in literature or from commercially available products. The donors and acceptors may be selected from the group consisting of fluorescent dyes and fluorescent proteins. Examples thereof include fluorescein derivatives, rhodamine derivatives, aminocoumarin derivatives, hydroxycoumarin derivatives, BODIPY derivatives, anthracene derivatives, benzofuran derivatives, porphyrin derivatives, CY dyes, ALEXA FLUOR dyes, europium cryptates, Dylight dyes, HILYTE FLUOR dyes, OYSTER dyes, MEGASTOKES dyes, IRDYEs, QSYs, DFPs, YFPs, GFPs and BFPs, and azamethine compounds such as those described in JP-ANo. 2001-089482.

When the present invention utilizes a fluorescence resonance energy transfer technique, fluorescent substances used in the invention are preferably stable substances between which an energy transfer may occur. Preferable examples thereof include a combination of a fluorescent protein and a fluorescent dye, such as a combination of CFP and YFP, a combination of ALEXA FLUOR 555 and ALEXA FLUOR 647, a combination of FITC and ALEXA FLUOR 555, and a combination of HILYTE FLUOR 555 and ALEXA FLUOR 647. Examples of fluorescent substances further include, but are not limited to, a combination of fluorescent proteins, a combination of fluorescent dyes, and a combination of a fluorescent dye and a fluorescent protein. In consideration of stability of fluorescence emission, the fluorescent substances are preferably ALEXA FLUOR dyes or HILYTE FLUOR dyes, and more preferably ALEXA FLUOR dyes.

In addition, fluorescence resonance energy transfer between CFP and YFP, between GFP and BFP, and between modified products of these dyes are well known, and these fluorescent proteins are commercially available. When the fluorescent protein is used, a single fusion protein in which an antibody to be labeled is linked with the fluorescent protein can be obtained. When the antibody is used in living cells, it is preferable to use a fluorescent protein since a fusion protein of antibody with the fluorescent protein can be obtained by introducing a gene encoding the fusion protein into the cells. Nucleic acids encoding such fluorescent proteins are well known and a variety of vectors containing such fluorescent proteins are commercially available. Therefore, a fluorescent labeled protein in which a fluorescent protein is fused with a desired polypeptide can be easily obtained by using those commercially available vectors.

Examples of the biological-luminescent donor substance with which BRET can occur include a luciferin derived from luminous organisms, such as *Photinus pyralis* luciferin, *Cypridina* luciferin, *Renilla reniformis* luciferin, *Diplocardia* luciferin, *Latia neritoides* luciferin, *Wataseniae* luciferin or bacterial luciferins (for example, reduced flavin mononucleotide); and aequorin derived from *Aequorea victoria*. Examples of the acceptor substances include the same fluorescent proteins and fluorescent dyes as those of acceptor substances used in FRET described above.

Mechanisms for accepting and detecting emission by FRET or BRET are known in the art. For example,

luminescence can be detected by using a method disclosed in BIOPHYSICS (Seibutsu Butsuri) Vol.42(1), pp.28-31 (2002). In this method, FRET or BRET can be confirmed by measuring and detecting luminescence obtained by irradiating light to a donor substance or inducing an enzyme reaction of a donor substance. In accordance with this method, antigen binding to antibodies immobilized on the immobilization substrate can be detected.

(3) A method for producing the antibody-fragment-immobilizing substrate

A method for producing an antibody-fragment-immobilizing substrate comprising:

contacting separate antibody fragments of at least two types that include at least one type of labeled antibody fragment having a labeled site labeled with a luminescent substance and at least one type of acceptor antibody fragment having an acceptor site for accepting emission from the labeled antibody fragment and that are capable of cooperatively recognizing one type of antigen, with the antigen, and forming a complex whereby each of the antibody fragments binds to the antigen;
immobilizing the complex on a substrate via the antibody fragments in the complex; and
removing the antigen from the complex to obtain the antibody-fragment-immobilizing substrate, wherein the antibody fragments are independently immobilized on the substrate in a positional relationship that allows each of the antibody fragments to bind to the same antigen.

According to the production method of the invention, since the complex composed of the antibody fragments of at least two types and the antigen is immobilized on the substrate, and then the antigen is removed from the complex, the antibody-fragment-immobilizing substrate, in which the antibody fragments are independently immobilized on the substrate in a positional relationship that allows each of the antibody fragments to bind to the same antigen in the presence of the antigen, can be easily produced.
According to the production method of the invention, an antibody-fragment-immobilizing substrate including a substrate and at least one set of (for example two or more sets of) antibody fragments may be obtained, in which the antibody fragments of each set include the at least two types of separate antibody fragments that have been immobilized on the substrate as described above.
When forming the complex, the complex composed of the antibody fragments and the antigen can be formed by a known technique. More specifically, the complex is easily obtained by mixing the above-described antibody fragments and antigen.
The mixing ratio of antibody fragments of at least two types to the antigen can be appropriately set depending on a binding mode of an antibody toward an antigen. In consideration of efficiency and in order to prevent excess immobilization of the antigens, the ratio of the number of antigens to the valencies of molecules formed by a combination of antibody fragments is from 0.1:1 to 10:1.
The mixing ratio of the antibody fragments and the antigen can be appropriately adjusted depending on affinity of the antibody fragments for an antigen, immobilization efficiency of the antigen per se to the substrate directly, or the like. In general, when the antigen is expected to have a sufficient affinity for the antibody fragments, the ratio of the number of antigens to the valencies of molecules formed by a combination of antibody fragments is from 0.1:1 to 1:1, and more preferably from 0.1:1 to 0.2:1. On the other hand, when the antigen is expected to have a low affinity or expected to be difficult to immobilize on the substrate directly, for example, when a low molecular compound or the like is used as the antigen, it is preferable to use an increased amount of the antigen. More specifically, the ratio of the number of antigens to the valencies of molecules formed by a combination of antibody fragments is preferably from 0.5:1 to 5:1.
Here, "the valencies of molecules formed by a combination of antibody fragments" means the number of the antigen-binding sites present in the molecules formed by a combination of antibody fragments. That is, when a molecule formed by a combination of antibody fragments constitutes a complete antibody molecule, the valencies of the molecules are equal to that of the antibody molecules. When a molecule formed by a combination of antibody fragments does not constitute a complete antibody molecule, the valency thereof is considered to be 1 as long as one antigen-binding site is included therein.
The antigen may be a low molecular weight molecule, or a high molecular weight molecule such as a protein. Although the complex may be formed by any number of molecules, in order to facilitate the control of the quantitative ratio, the number of molecules forming the complex is preferably three molecules such as two types of the antibody fragments and one antigen.
For example, when an anti-lysozyme VH-region polypeptide, an anti-lysozyme VL-region polypeptide and lysozyme (an antigen) are used, since the VH-region polypeptide and the VL-region polypeptide each interact with the antigen in a ratio of 1:1, a molecule formed by the combination of the VH-region polypeptide and the VL-region polypeptide has a valency of 1. Therefore, the complex can be readily obtained by mixing the VH-region polypeptide, VL-region

polypeptide, and the antigen in an equal number ratio in an aqueous solution, that is, in a ratio of 1:1:1. In order to prevent decreasing of binding efficiency to the antibody fragments caused by the direct immobilization of lysozyme on the substrate, the number ratio of the antigen is preferably less compared with the valency of the antibody fragment. The mixing ratio among the VH-region polypeptide, VL-region polypeptide, and the antigen is preferably from 10:10:1 to 10:10:9, and more preferably from 10:10:1 to 10:10:2.

The ratio of the amount of the labeled antibody fragment to the acceptor antibody fragment can be appropriately adjusted depending on affinity of the antibody fragments for an antigen. In general, the ratio of the amount of the labeled antibody fragment to the acceptor antibody fragment is 1:1 in order to form an antibody molecule having a valency of 1. In consideration of immobilization efficiency of antigen fragments after labeling, the ratio of the labeled antibody fragment labeled with a donor substance to the acceptor antibody fragment labeled with an acceptor substance is preferably from 15:1 to 1:15, more preferably from 10:1 to 1:10, and still more preferably from 5:1 to 1:5.

When immobilizing the complex, the complex formed by the procedure described above is linked to the substrate by conducting the reaction appropriately in accordance with the type of functional group provided to the substrate. In this case, since the antigen-recognition site of the antibody fragment is protected by binding of the antigen, no additional protective treatment is required. Methods for linking the complex to the substrate are well known by those skilled in the art. Examples thereof include a method of activating a carboxyl group using EDC described above or the like and linking to a complex via an amino group, or a method in which a complex is linked to the substrate by a reaction of a maleimide group with a thiol group, but the present invention is not restricted thereto.

When the immobilization substrate according to the present invention is used for a biosensor, the antibody fragment may be immobilized on the substrate in an amount (density) such that there is a sufficient distance between the labeled antibody fragment and the acceptor antibody fragment, for example, about 10 nm or less, in order for the acceptor antibody fragment to accept the luminescence. The immobilizing amount of the antibody fragment is preferably from $1\times10^5/mm^3$ to $1\times10^{18}/mm^3$, more preferably from $1\times10^9/mm^3$ to $1\times10^{15}/mm^3$, and still more preferably from $1\times10^{10}/mm^3$ to $1\times10^{14}/mm^3$. When the immobilizing amount is $1\times10^5/mm^3$ or more, the luminescence can be easily measured. When the immobilizing amount is $1\times10^{18}/mm^3$ or less, an appropriate distance between fluorescent substances can be kept and a good S/N ratio can be obtained in FRET. An immobilizing amount within the above range is also preferable from the viewpoint of the production cost.

The density can be determined by the following method. When the density is determined by actual measurement, each antibody fragment is immobilized on the substrate, and then a test substance is washed away. The density is calculated based on the labeling amount of the labeled antibody fragment and the emission accepting amount of the acceptor antibody fragment, and a film thickness measured by using an AFM or an ellipsometry technique. When the luminescent substance on the labeled antibody fragment and the emission-accepting substance on the acceptor antibody fragment are both fluorescence molecules, the fluorescence amount of each substance is measured by a fluorescence meter, and the number of immobilized molecules is determined based on a labeling ratio of fluorescence molecules obtained before immobilization, and the volume of the immobilized molecules is measured, thereby calculating the immobilization density.

When removing the antigen from the complex, the complex is immobilized on the substrate and then the antigen is removed from the complex. Since each of the antibody fragments is independently immobilized on the substrate, the antigen can be readily removed. Therefore, when the substrate is used as the immobilization substrate, binding reproducibility of the antigen may not decrease.

The removal of the antigen is readily carried out by using an appropriate washing solution. Any solution can be used as the washing solution as long as the avidity of the antigen and the antibodies in the complex is reduced. Examples of a condition for reducing the avidity include altering a pH toward an acidic side or an alkali side, and/or increasing a salt concentration. The washing solution varies depending on the type of the antibody fragment and antigen or the like, and examples thereof include an acidic glycine buffer with which the pH may be adjusted to 2 or less; an alkaline NaOH solution with which the pH may be adjusted to 10 or more; and a borate buffer with which the salt concentration may be adjusted to 0.5 M or more.

In addition, an acidic buffer containing arginine, a buffer containing guanidine or a buffer containing urea can be appropriately used.

Here, the condition of the washing treatment with the washing solution can be appropriately controlled. In order not to impair the activity of the antibody fragment, the time for the washing treatment is in general 10 minutes or less, and preferably 1 minute or less. From the viewpoint of reproducibility, the time for the washing treatment is preferably 5 seconds or more.

Accordingly, the immobilization substrate on which the antibody fragments are independently immobilized in a positional relationship that allows each of the antibody fragments in one set to bind to the same antigen can be obtained. In the immobilization substrate of the invention, the antibody fragments independently immobilized on the substrate have a degree of freedom and can bind to the antigen. That is, the labeled antibody fragment and acceptor antibody fragment in one set are independently immobilized on the substrate in a positional relationship similar that when they are bound

to the antigen. The antibody may be bound to the substrate via a part of the antibody, and a portion of the antibody having the antigen recognition site may be allowed to freely move. As long as the antibody fragments in one set can bind to one antigen, the antibody fragments after immobilization may freely move according to a position on the substrate where each antibody fragment binds (binding point) or the type of the substrate having the binding point (for example, the type of hydrophilic polymer).

Accordingly, when the antigen approaches the antibody fragments and may be bound, the antibody fragments may approach one another to bind to the antigen. In the present invention, the immobilization substrate for repeated use in which the antigen concentration can be determined rapidly by measuring an interaction between the luminescent substance and the accepting substance that accepts the luminescence, which are labeled with the respective antibody fragments, can be easily obtained.

For example, Figures 1A and 1B show an immobilization substrate 10, which is an example of the antibody-fragment-immobilizing substrate according to the present invention. In the immobilization substrate 10, antibody fragments 18A and 18C are independently immobilized on a substrate 12 via a self-assembled monolayer 14 and a polymer layer 16 through a part of each antibody. The antibody fragment 18A has a site labeled with a luminescent substance 18B, and the antibody fragment 18C has a luminescence accepting site 18D. The antibody fragment 18A and the antibody fragment 18C are bound to the substrate 12 through one end of each antibody fragment such that the other end of each antibody fragment can move freely, and are independently immobilized on the substrate 12 in a positional relationship that allows the antibodies to cooperatively bind to an antigen Ag if present (see Figure 1A).

When the antigen Ag approaches the immobilization substrate 10, the antibody fragments 18A and 18B recognize and bind to the antigen Ag. When binding, the antibody fragments 18A and 18C approach each other centering around the antigen and bind to the antigen, whereby the luminescent substance 18B on the antibody fragment 18A and the luminescence accepting site 18D on the antibody fragment 18C approach and interact. By measuring the change caused by this interaction, the antigen can be detected (see Figure 1B).

As described above, by immobilizing the complex formed by two or more antibody fragments and one or more antigens on the substrate through the two or more antibody fragments and then removing only the antigens, the antibody-fragment-immobilizing substrate which can be utilized in an immunoassay using an antigen-antibody reaction can be obtained.

Since the antibody fragments are independently immobilized on the substrate as described above, when the affinity between them is low, the antibody fragments do not associate with one another and can easily move independently of one another, whereby the conformational change of the antibody fragment more easily occurs when the antigen binds. This conformational change increases the efficiency of the fluorescent energy transfer. Regarding the affinity between the antibody fragments, the dissociation constant (Kd) is preferably from $10^{-12}$ to $10^{8}$, more preferably from $10^{-7}$ to $10^{8}$, and still more preferably form $10^{-5}$ to $10^{-1}$.

(II) Application of the immobilization substrate according to the present invention

The immobilization substrate according to the present invention can be applied to a biosensor or a bioreactor based on a binding reactivity between the antibody fragments and the antigen (for example, see "Bioreactor Technique", 1988, CMC Publishing Co., Ltd., and "Biochip and Biosensor", 2006, Kyoritsu Shuppan Co., Ltd.). Here, the bioreactor refers to a reactor applied for production of useful materials, generation of energy, degradation of environmental pollutants or the like by utilizing a biochemical reaction by biological catalysts such as enzymes, bacteria cells, cells, or organelles. Here, the term biosensor is intended to be interpreted most broadly, and means a sensor that measures and/or detects a target substance by converting an interaction between biological molecules into a signal such as an electrical signal. Hereinafter, the application to the bioreactor and the biosensor will be described.

(1) Application to the bioreactor

A bioreactor capable of carrying out generation of useful substances, reaction, or the like using an insoluble substrate immobilizing an enzyme is described (for example, Examined utility model application publication (JP-Y) Nos. 4-18398 and 4-18399). The substrate according to the present invention, for example, in the form of a substrate having a support (for example, a porous material such as ceramic or polysulfone), a polymer membrane bound to this substrate surface, and an enzyme and an auxiliary, substance for an enzyme activity which are bound to this polymer membrane can be applied, as such insoluble substrate, to the bioreactor.

(2) Application to a biosensor

In general, a biosensor is composed of a receptor portion recognizing target chemicals to be detected and a transducer portion converting a physical change or a chemical charge generated at the receptor portion into an electric

signal. Examples of a combination of substances having a mutual affinity in a living body include a combination of enzyme and substrate, a combination of enzyme and coenzyme, a combination of antigen and antibody, and a combination of hormone and receptor. The biosensor employs a principle that, by immobilizing one of the substances with mutual affinities of such a combination on a substrate and using it as a molecule-recognizing substance, the corresponding other substance is selectively measured. The substrate according to the present invention can be applied to be, for example, a substrate having a support (for example, a porous material such as ceramic or polysulfone), a polymer membrane bound to this substrate surface, and two types of antibody fragments which are bound to this polymer membrane, in order to achieve more rapid detection compared with conventional biosensor and to obtain a biosensor that can be repeatedly used and have a greater versatility.

Hereinafter, the immobilization substrate of the invention will be described in more detail with reference to examples, but the invention is not limited to the examples. Further, "parts" and "%" indicate quantities in terms of mass, unless otherwise specified.

## EXAMPLES

### Example 1

(1) Preparation of anti-lysozyme VH-region polypeptide and anti-lysozyme VL-region polypeptide

Abbreviations used in the Examples are as follow:

- LB: culture medium containing 1% BACTO (Registered tradename) Tryptone, 0.5% Yeast Extract and 0.5% NaCl
- LBA: LB containing 100 μg/ml of ampicillin
- LBAG: LB containing 100 μg/ml of ampicillin and 0.1% glucose
- LBAG plate: LB agar medium containing 100 μg/ml of ampicillin and 0.1 % glucose
- SOC: culture medium containing 2% BACTO (Registered tradename) Tryptone, 0.5% Yeast Extract, 0.05% NaCl, 2.5 mM KCl, 20mM glucose and 10 mM $MgCl_2$
- PBS: 10 mM phosphate buffer (pH 7.2) containing 137 mM NaCl and 2.7 mM KCl
- 5% IBPBS: PBS containing 5% (v/v) of IMMONOBLOCK (trade name, manufactured by Dainippon Sumitomo Parma Co., Ltd., Osaka)
- 20% IBPBS: PBS containing 20% (v/v) IMMONOBLOCK
- PBST: PBS containing 0.1% of Triton-X 100
- TAE buffer: 40mM Tris-acetate buffer (pH 8.3) containing 1 mM EDTA
- TALON buffer: 50 mM sodium phosphate buffer (pH 7.0) containing 300 mM NaCl
- TALON elution buffer: TALON buffer (pH 7.0) containing 500 mM imidazole
- IPTG: isopropyl-β-thiogalactopyranoside
- HBS-N buffer: 10 mM HEPES, 150 mM NaCl, pH 7.4

In all experiments, water purified with MILLI-Q (trade name, manufactured by Millipore Co., Billerica, MA, USA) was used. Hereinafter, this purified water is referred to as milliQ water. Unless otherwise specified, general reagents used were obtained from Sigma-Aldrich Co. (St. Louis, MO, USA), Nacalai Tesque (Kyoto, Japan), Wako Pure Chemical Industries, Ltd. (Osaka, Japan), or Kanto Chemical Co. Inc. (Tokyo, Japan). Oligo DNAs were synthesized by Texas Genomics Japan (Tokyo, Japan) or Invitrogen (Tokyo, Japan).

The genotypes of E. coli XL10-Gold and OverExpress C41 are shown in Table 1, and the primer sequences used for PCR are shown in Table 2.

Table 1

<*E. coli.* >

XL10-Gold:

Tetr *Δ(mcrA) 183 Δ(mcrCB-hsdSMR-mrr) 173 endA1 supE44 thi-1 recA 1 gyrA96 relA1 lac* Hte [F'*proABlacIqZΔM15 Tn10* ($Tet^r$) Amy $Cam^r$]

OverExpress C41(DE3) :

$F^-$, *omp T*, *hsd*$S_B$($r_B^-$ $m_B^-$), *gal*(λ cI 857, *ind*1, *Sam*7, *nin*5, *lacUV5*-T7*gene*1), *dem*(DE3)

Table 2

<Primer>

(1) SEQ ID NO: 1

AAAAAAAGCGGCCGCGGAGCATCATCACCATCACCACCACCACCACCACT GAGATCCGG

(The underline indicates the NotI site and the double-underline indicates the His10 sequence.)

(2) SEQ ID NO: 2

CCAATGCTTAATCAGTGA

(3)SEQ ID NO:3

CTTTCTATGCGGCCCAGCCGGCCATGGCCGAKGTSVAGCTTCAGGAGTC

(The underline indicates the SfiI site.)

(4) SEQ ID NO: 4

AAAAAAGCGGCCGCGCTCGAGACGGTGACCGTGG

(The underline indicates the NotI site.)

(5) SEQ ID NO: 5

AAAAAAGGCCCAGCCGGCCATGGCGTCGACGGATATTTTGATGAC

(The underline indicates the SfiI site.)

(6) SEQ ID NO: 6

TTTCTCGTGCGGCCGCACGTTTTATTTCCAACTTTG

(The underline indicates the NotI site.)

(A) Construction of expression plasmids

(a) Vectors used in the experiments

- pET-MBPp-His6: pET15b vector (Merck Chemicals Ltd., Darmstadt, Germany), into which the gene encoding maltose binding protein (MBP) tagged with His-Tag containing six histidine residues (His6) is inserted (SEQ-IDNO: 7).
- pIT2-LxE16: pIT2 vector (provided by MRC Cambridge, UK), into which the single-chain antibody (scFv) gene encoding anti-hen egg lysozyme (HEL) antibody LxE16 (SEQ ID NO: 8, amino acid: SEQ ID NO: 9) (isolated at Laboratory of Protein Engineering, Department of Chemistry and Biotechnology, Graduate School of Engineering, University of Tokyo) is inserted.

(b) Outline of the preparation of the expression vectors

As shown in the scheme of Figure 2, each of an expression vector pET-MBPp-VH(HEL)-His10 encoding a MBP-VH(HEL)-His10 protein, in which MBP and a His-tag containing ten histidine residues (His 10) are respectively fused to N- and C-terminals of VH(HEL) (the heavy-chain variable region domain of the anti-lysozyme antibody LxE16), and an expression vector pET-MBPp-VL(HEL)-His10 encoding a MBP-VL(HEL)-His10 protein, in which MBP and His10 are respectively fused to N-and C-terminals of VL(HEL) (the light-chain variable region domain of the anti-lysozyme antibody LxE16) was constructed by using the pET-MBPp-His6.

First, DNA fragment (1) containing His6 was isolated from the pET-MBPp-His6, and then DNA fragment (2) encoding His10 was inserted thereinto, thereby obtaining a pET-MBPp-His10. Subsequently, a VH(HEL) gene (SEQ ID NO: 10, Table 3) was inserted into the pET-MBPp-His10, thereby obtaining the pET-MBPp-VH(HEL)-His10. Separately, a VL(HEL) gene (SEQ ID NO: 11, Table 4) was inserted into the pET-MBPp-His10, thereby obtaining the pET-MBPp-VL(HEL)-His10.

Table 3

VH(HEL)

ATGGCCGAGGTGCAGCTTCAGGAGTCAGGACCTAGCCTCGTGAAACCTTCTCAG
ACTCTGTCCCTCACCTGTTCTGTCACTGGCGACTCCATCACCAGGGGTTACTGGA
GCTGGATCCGGAAATCCCCAGGAAATAAACTTGAGTACATGGGGTACATAAGCTA
CAGTGGTAGCACTTTCTACAATCCATCTCTCAAAAGTCGAATCTCCATCACTCGAG
ACACATTCAAGAACCAGCTCTACCTGCAGTTGAATTCTGTGACTACTGAGGACACA
GCCACATATTATTGTGCAGAGTACGACGGGACTTACTGGGGCCAAGGGACCACG
GTCACCGTCTC

Table 4

VL(HEL)

TCGACGGATATTTTGATGACCCAGACTCCAGCCACCCTGTCTGTGACTCCAGGAGA
TAGGGTCAGTCTTTCCTGCAGGGCCAGCCAAAGTATTGGCAACAACCTACACTGGT
TTCAACAAAAATCACATGAGTCTCCAAGGCTTCTCATCAAGTATGCTTCCCAGTCCA
TCTCTGGGATCCCCTCCAGGTTCAGTGGCAGTGGATCAGGGACAGATTTCACTCTC
AGTATCAACACTGTGGAGACTGAAGATTTTGGAATGTATTTCTGTCAACAGAGTAAC
AGCTGGCCGTACACGTTCGGAGGGGGGACAAAGTTGGAAATAAAACGT

(b) Isolation of DNA fragment (1) from the pET-MBPp-His6

To 74 μl of an aqueous solution containing about 10 μg of pET-MBPp-His6, 3 μl of ScaI (Roche Applied Science, Basel, Switzerland, 10 U), 3 μl of NotI (Roche Applied Science, 10 U), 10 μl of 10×BSA solution and 10 μl of 10×H buffer (Roche Applied Science) were added, and the mixture was then left to stand for about 3 hours at 37°C. Subsequently, the mixture was subjected to electrophoresis on 1% agarose gel (in TAE buffer), and then a DNA band of approximately 4,100 bp was excised and extracted using WIZARD SV Gel And PCR Clean-Up System (trade name, manufactured by Promega Co., Madison, WI). The extracted DNA was dissolved in 50 μl of milliQ water.

(c) Preparation of DNA fragment (2)

PCR was performed by using the pET-MBPp-His6 as the template, a primer (1) (SEQ ID NO: 1) and a primer (2) (SEQ ID NO: 2). The primer (1) is a reverse primer having a nucleic acid sequence corresponding to ten histidine residues and having the NotI site; and an annealing site of the primer (1) is located downstream of the His6 coding region. The primer (2) is a forward primer; and an annealing site of the primer (2) is located approximately 500 bases downstream of the ScaI site of the pET vector.
The PCR conditions are as follows:

Composition of the reaction mixture

| | |
|---|---|
| pET-MBPp-His6 (about 100 μg/ml) | 0.5 μl |
| Primer (1) (50 μM) | 0.5 μl |
| Primer (2) (50 μM) | 0.5 μl |
| 10×Pfu buffer (20 mM $Mg^{2+}$) (Agilent Technologies, Inc., Santa Clara, CA) | 5 μl |
| dNTP Mixture (2.5 mM each) | 4 μl |
| 2.5 U/μl of Pfu DNA polymerase (Agilent Technologies, Inc.) | 0.5 μl |
| milliQ water | 39 μl |

Reaction cycle

1. 94°C, 1 min
2. 94°C, 30 sec
3.58°C, 30 sec
4. 72°C, 30 sec

(25 cycles of steps 2 to 4)
5. 72°C, 10 min
6. 16°C ∞

The PCR product was purified with WIZARD SV Gel And PCR Clean-Up System (Promega Co., Madison, WI) and dissolved in 50μl of milliQ water. To the solution, 1 μl of ScaI (Roche Applied Science, 10 U), 1 μl of NotI (Roche Applied Science, 10 U), 7 μl of 10×BSA solution, 7 μl of 10×H buffer (Roche Applied Science) and 4 μl of milliQ water were added, and the mixture was then left to stand for about 3 hours at 37°C. Subsequently, the mixture was subjected to electrophoresis on 1% agarose gel (in TAE buffer), and then a DNA band of approximately 1,080 bp was excised and extracted using WIZARD SV Gel And PCR Clean-Up System. The extracted DNA was dissolved in 50 μl of milliQ water, thereby obtaining a solution of DNA fragment (2).

(d) Preparation ofpET-MBPp-His10

0.5 μl of a solution containing the pET-MBPp-His6 from which DNA fragment (1) has been removed was mixed with 5 μl of the solution of DNA fragment (2). Subsequently, 5.5 μl of DNA LIGATION HIGH Ver2 Solution (trade name, manufactured by TOYOBO Co., Ltd., Osaka, Japan) was added to the mixture, and then DNA ligation was performed for 30 minutes at 16°C. Thereafter, about 50 μl of E. coli XL10-Gold chemical competent cells were transformed with about 1 μl of the ligation reaction mixture. The transformants were cultured on LBAG agar medium overnight at 37°C. A single-colony transformant was further cultured in 50 ml of LBAG overnight, and then the plasmid DNA was extracted using WIZARD PLUS MINIPREPS DNA Purification Kit (trade name, manufactured by Promega Co.), thereby obtaining a pET-MBPp-His10. The DNA sequence encoding His10 was confirmed in accordance with a protocol from Beckman Coulter, Inc.

(e) Restriction enzyme treatment of pET-MBPp-His10

To 46 μl of an aqueous solution containing about 7 μg of pET-MBPp-His10, 2 μl of SfiI (Roche Applied Science, 10 U), 6 μl of 10×BSA solution and 6 μl 10×M buffer (Roche Applied Science) were added, and the mixture was then left to stand for about 3 hours at 50°C. DNA was purified using WIZARD SV Gel And PCR Clean-Up System and then dissolved in 50 μl of an aqueous solution. To the DNA solution, 2 μl of NotI (Roche Applied Science, 10 U), 7 μl of 10xBSA solution, 7 μl of 10xH buffer (Roche Applied Science) and 4 μl of milliQ water were added, and the mixture was then left to for about 3 hours stand at 37°C. Subsequently, the mixture was subjected to electrophoresis on 1% agarose gel (in TAE buffer), and then a DNA band of approximately 4,800 bp was excised and extracted using WIZARD SV Gel And PCR Clean-Up System. The extracted DNA was dissolved in 50 μl of milliQ water.

(f) Preparation of VH(HEL) gene fragment

PCR amplification of the VH(HEL) gene fragment was performed by using pIT2-LxE16 as a template, and a primer (3) and a primer (4). The primer (3) is a reverse primer having a SfiI site; and an annealing site of the primer (3) is located at the 5' side of the VH(HEL) gene fragment. The primer (4) is a forward primer having the NotI site; and an annealing site of the primer (4) is located at the 3' side of the VH(HEL) gene fragment.

The PCR conditions are as follows:

Reaction mixture composition

| | |
|---|---|
| pIT2-LxE16 (about 100 μg/ml) | 0.5 μl |
| Primer (3) (50 μM) | 0.5 μl |
| Primer (4) (50 μM) | 0.5 μl |
| l0xPfu buffer (20 mM $Mg^{2+}$) (Agilent Technologies, Inc.) | 5 μl |
| dNTP Mixture (2.5 mM each) | 4 μl |
| 2.5 V/μl of Pfu DNA polymerase (Agilent Technologies, Inc.) | 0.5 μl |
| milliQ water | 39 μl |

Reaction cycle

1. 94°C, 1 min
2. 94°C, 30 sec

3. 58°C, 30 sec
4. 72°C, 30 sec
(25 cycles of steps 2 to 4)
5. 72°C, 10 min
6. 16°C ∞

The PCR product was purified with WIZARD SV Gel And PCR Clean-Up System and dissolved in 50 μl of milliQ water. To the solution, 2 μl of SfiI (Roche Applied Science, 10 U/μl), 7 μl of 10xBSA solution, 7 μl of 10xM buffer (Roche Applied Science) and 4μl of milliQ water were added, and the mixture was then left to stand for about 3 hours at 50°C. The resulting DNA was purified with WIZARD SV Gel And PCR Clean-Up System and dissolved in 50 μl of aqueous solution. To the DNA solution, 2 μl of NotI (Roche Applied Science, 10 U), 7μl of 10xBSA solution, 7μl of 10xH buffer (Roche Applied Science) and 4 μl of milliQ water were added, and the mixture was then left to stand for about 3 hours at 37°C. Subsequently, the resulting DNA was purified with WIZARD SV Gel And PCR Clean-Up System. The extracted DNA was dissolved in 50 μl of milliQ water, thereby obtaining a solution of the VH(LxE16) gene fragment.

(g) Preparation of VL(HEL) gene fragment

PCR amplification of the VL(HEL) gene fragment was performed by using pIT2-LxE16 as a template, and a primer (5) and a primer (6). The primer (5) is a reverse primer having a SfiI site; and an annealing site of the primer (5) is located at the 5' side of the VL(HEL) gene fragment. The primer (6) is a forward primer having a NotI site; and an annealing site of the primer (6) is located at the 3' side of the VL(HEL) gene fragment. PCR, restriction enzyme treatment and purification of DNA were performed in the same manner as in preparation of VH(LxE16) gene fragment, thereby obtaining a solution of the VL(LxE16) gene fragment.

(h) Preparation of pET-MBPp-VH(HEL)-His10 and pET-MBPp-VL(HEL)-His10

0.5 μl of a solution containing the restriction enzyme treated pET-MBPp-His10 was mixed with 5 μl of the solution of VH(LxE16) or VL(LxE16). Subsequently, 5.5 μl of DNA LIGATION HIGH Ver2 Solution (TOYOBO Co., Ltd.) was added to the mixture, and then DNA ligation was performed for 30 minutes at 16°C. Thereafter, about 50 μl of E. coli XL10-Gold chemical competent cells were transformed with about 1 μl of the ligation reaction mixture. The transformants were cultured on LBAG agar medium overnight at 37°C. Single colony transformants were further cultured in 50 ml of LBAG overnight, and then the plasmid DNA was extracted using WIZARD PLUS MINIPREPS DNA Purification Kit (Promega Co.). The DNA sequences of pET-MBPp-VH(HEL)-His10 and pET-MBPp-VL(HEL)-His10 were confirmed in accordance with a protocol from Beckman Coulter, Inc.

(B) Preparation of MBP-VH(HEL)-His10 protein and MBP-VL(HEL)-His10 protein

The pET-MBPp-VH(HEL)-His10 and pET-MBPp-VL(HEL)-His10 plasmids were respectively transformed into E. coli OverExpress C41(DE3) by the heat shock method to express the genes. 1 μl of the plasmid (about 100 ng) and 100 μl of OverExpress C41(DE3) competent cells were mixed, and the mixture was then left to stand for 30 min on ice. Subsequently, heat shock was performed for 45 seconds at 42°C. Immediately after the heat shock, the mixture was left to stand for 2 minutes on ice. Thereafter, the cells were cured for 30 minutes by adding 200 μl of SOC medium thereto. The mixture was then spread on LBA plate and incubated overnight at 37°C.

A grown colony was inoculated into 4 ml of LBAG and cultured overnight at 30°C with shaking. 4 ml of the small-scale culture was then added to 800 ml of LBA, and cultured at large scale at 30°C with shaking. When an O.D.600 of the culture reached between 0.5 and 0.6,400 μl of 1000 mM IPTG was added thereto and further cultured overnight at 30°C with shaking. The bacterial culture was then separated into supernatants and pellets of E. coli by centrifugation. By the methods described below, the MBP-VH(HEL)-His10 protein (SEQ ID NO: 12, Table 5) was independently collected from the supernatants by ammonium sulfate precipitation and from the pellet by ultrasonication of bacterial cells. Further, by the methods described below, the MBP-VL(HEL)-His10 protein (SEQ ID NO: 13, Table 6) was independently collected from the supernatants by ammonium sulfate precipitation and from the pellet by ultrasonication of bacterial cells.

Table 5

MBP-VH(HEL)-His10

MKIKTGARILALSALTTMMFSASALAKIEEGKLVIWINGDKGYNGLAEVGKKFEKDTGIKV
TVEHPDKLEEKFPQVAATGDGPDIIFWAHDRFGGYAQSGLLAEITPDKAFQDKLYPFTW
DAVRYNGKLIAYPIAVEALSLIYNKDLLPNPPKTWEEIPALDKELKAKGKSALMFNLQEPY
FTWPLIAADGGYAFKYENGKYDIKDVGVDNAGAKAGLTFLVDLIKNKHMNADTDYSIAE
AAFNKGETAMTINGPWAWSNIDTSKVNYGVTVLPTFKGQPSKPFVGVLSAGINAASPNK
ELAKEFLENYLLTDEGLEAVNKDKPLGAVALKSYEEELAKDPRIAATMENAQKGEIMPNI
PQMSAFWYAVRTAVINAASGRQTVDEALKDAQTNSSSNNNNNNNNNNLGPGAAHY--
VEFAAQPAMADVELQESGPSLVKPSQTLSLTCSVTGDSITRGYWSWIRKFPGNKLEYM
GYISYSGSTFYNPSLKSRISITRDTFKNQLYLQLNSVTTEDTATYYCAEYDGTYWGQGTT
VTVSSAAAEHHHHHHHHHH

**-: Protease cleavage site**

Table 6

MBP-VL(HEL)-His10

MKIKTGARILALSALTTMMFSASALAKIEEGKLVIWINGDKGYNGLAEVGKKFEKDTGIKVT
VEHPDKLEEKFPQVAATGDGPDIIFWAHDRFGGYAQSGLLAEITPDKAFQDKLYPFTWDA
VRYNGKLIAYPIAVEALSLIYNKDLLPNPPKTWEEIPALDKELKAKGKSALMFNLQEPYFT
WPLIAADGGYAFKYENGKYDIKDVGVDNAGAKAGLTFLVDLIKNKHMNADTDYSIAEAAF
NKGETAMTINGPWAWSNIDTSKVNYGVTVLPTFKGQPSKPFVGVLSAGINAASPNKELA
KEFLENYLLTDEGLEAVNKDKPLGAVALKSYEEELAKDPRIAATMENAQKGEIMPNIPQM
SAFWYAVRTAVINAASGRQTVDEALKDAQTNSSSNNNNNNNNNNLGPGAAHY--
VEFAAQPAMASTDILMTQTPATLSVTPGDSVSLSCRASQSIGNNLHWFQQKSHESPRLLI
KYASQSISGIPSRFSGSGSGTDFTLSINTVETEDFGMYFCQQSNSWPYTFGGGTKLEIKR
AAAEHHHHHHHHHH

-: **Protease cleavage site**

In the case of using the supernatant, 344 g of ammonium sulfate was added to about 800 ml of the culture supernatant and the mixture was stirred overnight at 4°C. Subsequently, an insoluble matter containing MBP-VH(HEL)-His10 or MBP-VL(HEL)-His10 was collected by centrifugation and the pellet was suspended in 30 ml of TALON buffer. In the case of using the pellet of E. coli, the pellet was suspended in 30 ml of TALON buffer and then subjected to ultrasonication, followed by centrifugation, to collect a supernatant containing MBP-VH(HEL)-His10 or MBP-VL(HEL)-His10. The supernatants was dialyzed against the TALON buffer. Each protein collected in TALON buffer was applied to a column (16 mm-diameter x about 15 mm-height) filled with a TALON affinity resin (trade name, manufactured by Clontech Laboratories, Inc., Mountain View, CA). Subsequently, TALON affinity resin, onto which the protein was adsorbed, was washed with the TALON buffer, and then a TALON elution buffer was added to elute MBP-VH(HEL)-His10 or MBP-VL(HEL)-His10. The purified protein was confirmed by SDS-PAGE. The buffer of the eluate was changed to HBS-N, and then glycerol was added thereto at a final concentration of 16%. The obtained solution was stored at -80°C.

(C) Preparation of VL(HEL)-His10

To 1 ml of the purified MBP-VL(HEL)-His10 solution (in HBS-N, about 1000 μg/ml), 20 μl of GENENASE solution (New England Biolabs, Inc., Ipswich, MA) was added. The mixture was allowed to react at room temperature for about 5 hours. After the reaction, in the same manner as described above, VL(HEL)-His10 was purified using the TALON affinity resin and the buffer of the eluate was changed to HBS-N.

(D) Preparation of MBP-VH(HEL)-His10 labeled with ALEXA FLUOR 555

440 μl of a solution containing the MBP-VH(HEL)-His10 obtained above (in HBS-N, about 430 μg/ml) was labeled with ALEXA FLUOR (registered trademark) 555 (manufactured by Molecular Probes). The labeling reaction was performed at pH 7.0.

(E) Preparation of VL(HEL)-His10 labeled with ALEXA FLUOR 647

27 μl of a solution containing the VL(HEL)-His10 obtained above (in HBS-N, about 1,679 μg/ml) was labeled with ALEXA FLUOR (registered trademark) 647 (manufactured by Molecular Probes). The labeling was performed at pH 7.0.

(2) Fabrication of a substrate provided with a carboxyl group

(A) Washing the substrate

A glass slide (manufactured by Matsunami Glass Ind., Ltd) was irradiated with UV, and immersed in acetone and ultrasonicated for 5 minutes. Subsequently, the glass slide was removed from acetone and washed with purified water.

(B) Providing an amino (APS) group

72 ml of ethanol and 8 ml of purified water were mixed in a 100 ml glass vial. The mixed solution was heated for about 15 minutes at 60°C in an incubator (manufactured by AS ONE Corporation). To the heated mixed solution of ethanol and purified water, 80 μl of APS (γ-aminopropyltriethoxysilane) (manufactured by Tokyo Chemical Industry Co., Ltd.) (0.1% v/v) was added and dissolved. Subsequently, the washed glass slide was immersed in this solution and allowed to react in the incubator (manufactured by AS ONE Corporation) for 15 minutes at 60°C, thereby obtaining a glass slide provided with the amino group. After the reaction, the glass slide was removed from the solution, and then washed by immersing the glass slide into a solution of ethanol and water at a ratio of 9:1 and then removing it. The washing was repeated three times. The glass slide after the washing was dried by blowing nitrogen gas thereon and baked for 1 hour at 120°C. After the baking, the glass slide was washed by sequentially immersing it in ethanol and purified water.

(C) Active esterification of CMD

To a 0.5% by mass solution of CMD (carboxymethyl dextran, manufactured by Meito Sangyo Co., Ltd., molecular weight 1,000,000) dissolved in ultrapure water, a mixed solution containing 0.4 M EDC (1-(3-Dimethylaminopropyl)-3-ethylcarbodiimide) and 0.1 M NHS (N-Hydroxysuccinimide) was added, being an amount calculated such that 2% of the carboxyl group in CMD is activated when the entire amount was reacted, and stirred for 5 minutes at room temperature.

(D) Preparation of a CMD film

The active esterified CMD solution was dropped onto the above prepared glass substrate having the amino group thereon. After 30 seconds, the solution was removed, whereby a thin film of active esterified carboxy methyl dextran was formed on the substrate having the amino group. The substrate was reacted for 1 hour at room temperature, and then washed once with 0.1 N NaOH and once with ultrapure water.

(3) Formation of a complex

MBP-VH(HEL)-His10 labeled with ALEXA FLUOR 555 and VL(HEL)-His10 labeled with ALEXA FLUOR 647, both of which were prepared above, and lysozyme were mixed at a number ratio of 1:1:1 to prepare a 1.5 μM each solution in HEPES buffer. The mixed solution was left to stand for 3 hours at room temperature, thereby obtaining a complex sample solution.

(4) Immobilization of the complex

The CMD glass substrate prepared above was set in a surface prep unit of a surface plasmon resonance analyzer (trade name: BIACORE3000, manufactured by Biacore). Using the surface prep unit, 70 μl of a mixed aqueous solution of 0.2 mM EDC and 0.04 mM NHS was added to the glass substrate at a flow rate of 10 μl/min. Subsequently, 30 μl of a two-fold dilution of the above complex sample solution with an acetate buffer (pH 4.5) was injected onto the glass substrate, and then blocking treatment was performed with an ethanolamine solution to immobilize the complex.

(5) Washing of the antigen

After the immobilization of the complex, the substrate was subjected to 1-minute washing for five times by

alternately using a glycine buffer (pH 1.5) and 10 mM NaOH to remove the antigen, thereby obtaining an immobilization substrate A of Example 1. The fluorescence intensity of the substrate after washing was measured using FLA-8000 (trade name, manufactured by FUJIFILM Corporation) and the intensity ratio between the emission at approximately 570 nm and the emission at approximately 675 nm was determined.

(6) Evaluation of the change in the amount of the fluorescence by the antigen

1 μM of lysozyme was injected onto the immobilization substrate A obtained above over 1 minute, and then the substrate was washed with the HEPES buffer solution. Subsequently, the fluorescence intensity was measured by using FLA-8000 and the intensity ratio between the emission at approximately 570 nm and the emission at approximately 675 nm was determined. The intensity ratio between the emission at approximately 570 nm and the emission at approximately 675 nm obtained in this manner was divided by the intensity ratio between the emission at approximately 570 nm and the emission at approximately 675 nm obtained in section (5) above, thereby determining the change in the emission intensity ratio before and after the addition of lysozyme.

Example 2

The immobilization substrate A used in Example 1 was subjected to 1-minute washing for five times by alternately using a glycine buffer (pH 1.5) and 10 mM NaOH to remove the antigen, thereby obtaining an immobilization substrate B of Example 2.

The fluorescence intensity of the immobilization substrate B was measured using FLA-8000 (trade name, manufactured by FUJIFILM Corporation) and the intensity ratio between the emission at approximately 570 nm and the emission at approximately 675 nm was determined.

Subsequently, 1 μM of lysozyme was injected onto the immobilization substrate B over 1 minute, and then the substrate was washed with the HEPES buffer solution. The fluorescence intensity was then measured by using FLA-8000 and the intensity ratio between the emission at approximately 570 nm and the emission at approximately 675 nm was determined.

Example 3

An immobilization substrate C was prepared in the same manner as in Example 1, except that the mixing ratio among MBP-VH(HEL)-His10 labeled with ALEXA FLUOR 555, VL(HEL)-His10 labeled with ALEXA FLUOR 647 and lysozyme in formation of the complex was changed to 10:10:1. The intensity ratio between the emission at approximately 570 nm and the emission at approximately 675 nm of the immobilization substrate C before and after the addition of lysozyme was determined in the same manner as in Example 1.

Comparative Example 1

An immobilization substrate D was prepared in the same manner as in Example 1, except that, in formation of the complex, MBP-VH(HEL)-His10 labeled with ALEXA FLUOR 555 and VL(HEL)-His10 labeled with ALEXA FLUOR 647 were added to the substrate for an identical amount of time, in an alternating manner. The intensity ratio between the emission at approximately 570 nm and the emission at approximately 675 nm of the immobilization substrate D before and after the addition of lysozyme was determined in the same manner as in Example 1.

Determination of the change in the amount of the fluorescence before and after the addition of lysozyme

Based on the results obtained in Examples 1 to 3 and Comparative Example 1, the percentage of the change of the emission intensity ratio before and after the addition of lysozyme was determined based on the intensity ratio between the emission at approximately 570 nm and the emission at approximately 675 nm. The results are shown in Table 7.

Table 7

| | Immobilization substrate | Percentage of the change of the emission intensity ratio |
|---|---|---|
| Example 1 | A | 5% |
| Example 2 | B | 5% |
| Example 3 | C | 6% |
| Comparative Example 1 | D | 0% |

As is apparent from Table 7, in the immobilization substrate (Comparative Example 1), which was prepared without forming the complex between the antibody fragments and the antigen and in which the positions of the antibody fragments on the substrate are not controlled, the change of the emission intensity ratio was not observed. On the other hand, each of the immobilization substrates of Examples 1, 2 and 3 exhibits increase in the percentage of the change of the emission intensity, which indicates that the antibody fragments are located adjacent to each other such that fluorescence resonance energy transfer occurs between them. Therefore, according to the present invention, the reaction of a complex composed of three or more substances can be detected, and the detection sensitivity and accuracy can be improved.

In addition, the immobilization substrate of Example 3, in which the mixing ration among anti-lysozyme VH-region polypeptide, anti-lysozyme VL-region polypeptide and lysozyme is 10:10:1, exhibits the approximately the same percentage of the change of the emission intensity ratio as that of Example 1, which shows that the complex can be efficiently formed even when the mixing ratio thereof is not equal.

In Examples and Comparative Example, a difference in the percentages of the change of the emission intensity ratio before and after the addition of the same concentration of lysozyme is approximately 5%. However, in Comparative Example (immobilization substrate D), the change was not detected even when a 1000-fold concentration (1 mM) of lysozyme was used (data not shown). These results indicate that, although the test sample cannot be detected by using the immobilization substrate of Comparative Example, the immobilization substrates according to the present invention indicates the application of fluorescence resonance energy transfer to the solid-phase to detect samples.

Example 4

(1) Preparation of MBP-VL(HEL)-His10 labeled with HILYTE FLUOR 555

100 μl of a solution containing the MBP-VL(HEL)-His10 obtained above (in HBS-N, about 796 μg/ml) was labeled with HILYTE FLUOR (registered trademark) 555 (manufactured by Dojindo Laboratories). The labeling reaction was performed at pH 7.0.

(2) Preparation of MBP-VH(HEL)-His10 labeled with ALEXA FLUOR 647

300 μl of a solution containing the MBP-VH(HEL)-His10 obtained above (in HBS-N, about 150 μg/ml) was labeled with ALEXA FLUOR (registered trademark) 647 (manufactured by Molecular Probes). The labeling was performed at pH 7.0.

An immobilization substrate E of Example 4 was prepared in the same manner as in Example 1, except that the mixing ratio among MBP-VL(HEL)-His10 labeled with HILYTE FLUOR 555, MBP-VH(HEL)-His10 labeled with ALEXA FLUOR 647 and lysozyme in formation of the complex was changed to 10:10:2. The intensity ratio between the emission at approximately 570 nm and the emission at approximately 675 nm of the immobilization substrate E before and after the addition of 1 μM of lysozyme for 10 minutes was determined in the same manner as in Example 1.

Example 5

The immobilization substrate E used in Example 4 was subjected to 1-minute washing for five times by alternately using a glycine buffer (pH 1.5) and 10 mM NaOH to remove the antigen, thereby obtaining an immobilization substrate F of Example 5.

The fluorescence intensity of the immobilization substrate F was measured using FLA-8000 (trade name, manufactured by FUJIFILM Corporation) and the intensity ratio between the emission at approximately 570 nm and the emission at approximately 675 nm was determined.

Subsequently, 1 μM of lysozyme was injected onto the immobilization substrate F over 10 minutes, and then the substrate was washed with the HEPES buffer solution. The fluorescence intensity was then measured by using FLA-8000 and the intensity ratio between the emission at approximately 570 nm and the emission at approximately 675 nm was determined.

Example 6

The immobilization substrate F used in Example 5 was subjected to 1-minute washing for five times by alternately using a glycine buffer (pH 1.5) and 10 mM NaOH to remove the antigen, thereby obtaining an immobilization substrate G of Example 6.

The fluorescence intensity of the immobilization substrate G was measured using FLA-8000 (trade name, manufactured by FUJIFILM Corporation) and the intensity ratio between the emission at approximately 570 nm and the

emission at approximately 675 nm was determined.

Subsequently, 100 nM of lysozyme was injected onto the immobilization substrate G over 10 minutes, and then the substrate was washed with the HEPES buffer solution. The fluorescence intensity was then measured by using FLA-8000 and the intensity ratio between the emission at approximately 570 nm and the emission at approximately 675 nm was determined.

Comparative Example 2

An immobilization substrate H was prepared in the same manner as in Example 4, except that, in the immobilization of the complex, MBP-VL(HEL)-His10 labeled with HILYTE FLUOR 555 and MBP-VH(HEL)-His10 labeled with ALEXA FLUOR 647 were added to the substrate for an identical amount of time, in an alternating manner. The intensity ratio between the emission at approximately 570 nm and the emission at approximately 675 nm of the immobilization substrate H before and after the addition of lysozyme was determined in the same manner as in Example 4.

Determination of the change in the amount of the fluorescence before and after the addition of lysozyme

Based on the results obtained in Examples 4 to 6 and Comparative Example 2, the percentage of the change of the emission intensity ratio before and after the addition of lysozyme was determined based on the intensity ratio between the emission at approximately 570 nm and the emission at approximately 675 nm. The results are shown in Table 8.

Table 8

| | Immobilization substrate | The percentage of the change of the emission intensity ratio |
|---|---|---|
| Example 4 | E | 10% |
| Example 5 | F | 11% |
| Example 6 | G | 5% |
| Comparative Example 2 | H | 0% |

As is apparent from Table 8, even when MBP-VL was labeled with the donor HILYTE FLUOR 555 and MBP-VH was labeled with the acceptor ALEXA FLUOR 647, the antibody-fragment immobilization substrate according to the present invention can be obtained. Therefore, it is thought that a similar measurement can be performed when MBP-VH and MBP-VL are labeled with donor and acceptor dyes respectively, or when MBP-VH and MBP-VL are labeled with acceptor and donor dyes respectively.

In addition, the results of Examples 5 and 6 indicate that the antibody-fragment immobilization substrate of the present invention can be used for repeated detection of the antigen even after washing. Further, since the emission intensity depends on an antigen concentration, the concentration of the antigen can be quantified using the antibody-fragment immobilization substrate of the present invention.

Furthermore, in the antibody-fragment immobilization substrate of the present invention, a similar measurement can be performed when either one of VH(HEL)-His10 and VL(HEL)-His10 is formed as the MBP fusion product, or when both of them are formed as the MBP fusion products, which indicates that the measurement can be performed regardless of whether or not the antibody fragment is an MBP fusion product.

Example 7

(1) Preparation of a fusion protein of anti-osteocalcin (BGP) VH-region polypeptide (VH(BGP)) with MBP (MBP-VH(BGP)), and a fusion protein of anti-BGP VL-region polypeptide with MBP (MBP-VL(BGP))

Abbreviations used in the Examples below are as follow:

- LB: Same as that used in Example 1
- LBA: Same as that used in Example 1
- LBAG: Same as that used in Example 1
- LBA plate: Same as that used in Example 1
- SOC: Same as that used in Example 1
- PBS: 10 mM phosphate buffer (pH 7.2) containing 137 mM NaCl and 2.7 mM KCl

- 5% IBPBS: PBS containing 5% (v/v) IMMUNOBLOCK (trade name, manufactured by Dainippon Sumitomo Pharma Co., Ltd., Osaka)
- 20% IBPBS: PBS containing 20% (v/v) IMMUNOBLOCK
- PBST: PBS containing 0.1% of Triton-X 100
- TAE buffer: Same as that used in Example 1
- TALON buffer: Same as that used in Example 1
- TALON elution buffer: Same as that used in Example 1
- IPTG: Same as that used in Example 1
- HBS-N buffer: Same as that used in Example 1

Similarly to Examples 1 to 6, water purified with MILLI-Q (trade name, manufactured by Millipore Co., Billerica, MA) was used in all experiments. Hereinafter the purified water is referred to as milliQ water. Unless otherwise specified, general reagents used were obtained from Sigma-Aldrich Co. (St. Louis, MO, USA), Nacalai Tesque (Kyoto, Japan), Wako Pure Chemical Industries, Ltd. (Osaka, Japan), or Kanto Chemical Co. Inc. (Tokyo, Japan). Oligo DNAs were synthesized by Texas Genomics Japan (Tokyo, Japan) or Invitrogen (Tokyo, Japan).

The genotypes of the E. coli XL10-Gold, OverExpress C41, and HB2151 are shown in Table 9.

Table 9

XL-10 gold: *Tetr*$\Delta$(*mcrA*) *183,* $\Delta$(*mcrCB-hsdSMR-mrr*)*173, endA1, supE44, thi-1, recA1, gyrA96, relA1, lac, Hte[F'proAB, lacIq, Z*$\Delta$*M15, Tn10*(*Tetr*)*, Tn5*(*Kanr*)*, Amy*]

OverExpress C41 (DE3) : F-, *ompT, hsdS*B(rB- mB-), *gal*($\lambda$ cI 857, *ind*1, *Sam*7, *nin*5, *lacUV*5-T7*gene*1), *dcm*(DE3)

HB2151 : *K12d*(*lac-pro*),*thi/F'; pro A+B+, Lac IqZ dM15*)

(A) Construction of expression plasmid for MBP-VH(BGP)

(a) Vectors used in the experiments

- pET MBPp-His6: pET15b vector (provided by Merck Chemicals Ltd., Darmstadt, Germany), into which the gene encoding maltose binding protein (MBP) tagged with His-Tag containing six histidine residues (His6) is inserted (described above, SEQ ID NO: 7).
- pIT2-VH(BGP): pIT2 vector, into which the gene encoding a VH-region polypeptide of anti-BGP antibody (VH(BGP)) (SEQ ID NO: 14, Table 10, amino acid: SEQ ID NO: 15) is inserted.
- pMAL-VL(BGP): an expression vector for a fusion protein of a VL-region polypeptide of anti-BGP antibody KTM219 and MBP (MBP-VL(BGP)) (described in, Lim et al. Anal. Chem. 79, 6193(2007)).

Table 10

VH(BGP)

GAGGTACAGCTGGAGGAGTCTGGGGCTGAGTTTGTGAAGGCTGGGGCTTCAGT
GAAGCTGTCCTGCAAGACTTCTGGCTACACCTTCAACAACTACTGGATTCACTG
GGTCAAACAGAGCCCAGGACAAGGCCTTGAATGGATCGGAGAAATTGATCCCT
CTGATGGTTATTCTAACTACAATCAAAAATTCAAGGGCAAGGCCACATTGACTGT
GGACAAGTCCTCCAGCACTGCCTACATGCACCTCAACAGTCTGACTTCTGAGGA
CTCTGCGGTCTATTATTGTACAAGCAGCACTTCGGTAGGAGGTTCCTGGGGCCA
AGGGACCACGGTCACCGTCTCGAGC

(b) Restriction enzyme treatment of pET-MBPp-His6

To 77 μl of an aqueous solution containing about 10 μg of pET-MBPp-His6, 3 μl of NotI (Roche Applied Science, Basel, Switzerland, 10 U), 10 μl of 10xBSA solution and 10 μl 10xH buffer (Roche Applied Science) were add, and the mixture was then left to stand for about 3 hours at 37°C. Subsequently, DNA was purified using WIZARD SV Ge1 And

PCR Clean-Up System (trade name, manufactured by Promega Co., Madison, WI) and dissolved in 50 μl of milliQ water. To the solution, 3 μl of SfiI (Roche Applied Science, 10 U), 7 μl of 10×BSA solution and 7 μl of 10×M buffer (Roche Applied Science) were added, and the mixture was then left to stand for about 3 hours at 50°C. Subsequently, the mixture was subjected to electrophoresis on 1% agarose gel (in TAE buffer), and then a DNA band of approximately 4,100 bp was excised and extracted using WIZARD SV Gel And PCR Clean-Up System (Promega Co.). The extracted DNA was dissolved in 50 μl of milliQ water.

(c) Isolation of VH(BGP) gene fragment from the pIT2-VH(BGP)

To 77 μl of an aqueous solution containing about 10 μg of pIT2-VH(BGP), 3 μl of NotI (Roche Applied Science, 10 U), 10 μl of 10×BSA solution and 10 μl 10×H buffer (Roche Applied Science) were added, and the mixture was then left to stand for about 3 hours at 37°C. Subsequently, DNA was purified using WIZARD SV Gel And PCR Clean-Up System (Promega Co.) and the purified DNA was dissolved in 50 μl of milliQ water. To the DNA solution, 3 μl of SfiI (Roche Applied Science, 10 U), 7 μl of 10×BSA solution, and 7 μl of 10×M buffer (Roche Applied Science) were added and the mixture was then left to stand for about 3 hours at 50°C. Subsequently, the mixture was subjected to electrophoresis on 1% agarose gel (in TAE buffer), and then a DNA band of approximately 450 bp was excised and extracted using WIZARD SV Gel And PCR Clean-Up System (Promega Co.). The extracted DNA was dissolved in 50 μl of milliQ water.

(d) Incorporation of VH(BGP) into the pET-MBPp-His6

0.5 μl of a solution containing pET-MBPp-His6, which was treated with the restriction enzymes and purified as described above, and 5 μl of a solution of VH(BGP), which was treated with the restriction enzymes and purified as described above, were mixed. Subsequently, 5.5 μl of DNA LIGATION HIGH Ver2 Solution (TOYOBO Co., Ltd., Osaka) was added to the mixture, and then DNA ligation was performed for 30 minutes at 16°C. Thereafter, about 50 μl of E. coli XL10-Gold chemical competent cells were transformed with about 1 μl of the ligation reaction mixture. The transformants were cultured on a LBAG agar medium overnight at 37°C. A single-colony transformant was further cultured in 50 ml of LBAG overnight. The plasmid DNA was then extracted using WIZARD PLUS MINIPREPS DNA Purification Kit (Promega Co.), thereby obtaining pET-MBPp-VH(BGP). The DNA sequence of pET-MBPp-VH(BGP) was confirmed in accordance with a protocol from Beckman Coulter, Inc.

(B) Preparation of MBP-VH(BGP) protein and MBP-VL(BGP) protein

The pET-MBPp-VH(BGP) plasmid and the pMAL-VL(BGP) plasmid were respectively transformed into E. coli OverExpress C41(DE3) by heat shock method for the expression. 1 μl of the plasmid (about 100 ng) and 100 μl of OverExpress C41(DE3) competent cells were mixed, and the mixture was then left to stand for 30 min on ice. Subsequently, heat shock was performed for 45 seconds at 42°C. Immediately after the heat shock, the resultant was left to stand for 2 minutes on ice. Thereafter, the cells were cured for 30 minutes by adding 200 μl of SOC medium thereto. The mixture was then spread on an LBA plate and incubated overnight at 37°C.

A grown colony was inoculated into 4 ml of LBAG and cultured overnight at 30°C with shaking. 4 ml of the small-scale culture was then added to 800 ml of LBA, and cultured at large scale at 30°C with shaking. When an O.D.600 of the culture reached between 0.5 and 0.6, 400 μl of 1000 mM IPTG was added thereto and further cultured overnight at 20°C with shaking. The bacterial culture was then separated into supernatants and pellets of E. coli by centrifugation. By the methods described below, each of the MBP-VH(BGP) protein (SEQ ID NO: 16, Table 11) was independently collected from the supernatants by ammonium sulfate precipitation method and from the pellet by ultrasonication of bacterial cells. Further, the MBP-VL(BGP) protein (SEQ ID NO: 17, Table 12) was independently collected from the supernatants by ammonium sulfate precipitation method and from the pellet by ultrasonication of bacterial cells.

Table 11

MBP-VH(BGP)

MKIKTGARILALSALTTMMFSASALAKIEEGKLVIWINGDKGYNGLAEVGKKFEKDTGIKVT
VEHPDKLEEKFPQVAATGDGPDIIFWAHDRFGGYAQSGLLAEITPDKAFQDKLYPFTWD
AVRYNGKLIAYPIAVEALSLIYNKDLLPNPPKTWEEIPALDKELKAKGKSALMFNLQEPYFT
WPLIAADGGYAFKYENGKYDIKDVGVDNAGAKAGLTFLVDLIKNKHMNADTDYSIAEAAF
NKGETAMTINGPWAWSNIDTSKVNYGVTVLPTFKGQPSKPFVGVLSAGINAASPNKELA
KEFLENYLLTDEGLEAVNKDKPLGAVALKSYEEELAKDPRIAATMENAQKGEIMPNIPQM
SAFWYAVRTAVINAASGRQTVDEALKDAQTNSSSNNNNNNNNNNLGPGAAHYVEFAAQ
PAEVQLEESGAEFVKAGASVKLSCKTSGYTFNSYWIHWIKQSPGQGLEWIGEIDPSDGY
TNYNQKFKGKATLTVDKSSSTAYMRLNSLTSEDSAVYYCTSSTSVGGSWGQGTTVTVS
SHHHHHH

Table 12

MBP-VL(BGP)

MKIKTGARILALSALTTMMFSASALAKIEEGKLVIWINGDKGYNGLAEVGKKFEKDTGIKVT
VEHPDKLEEKFPQVAATGDGPDIIFWAHDRFGGYAQSGLLAEITPDKAFQDKLYPFTWDA
VRYNGKLIAYPIAVEALSLIYNKDLLPNPPKTWEEIPALDKELKAKGKSALMFNLQEPYFTW
PLIAADGGYAFKYENGKYDIKDVGVDNAGAKAGLTFLVDLIKNKHMNADTDYSIAEAAFNK
GETAMTINGPWAWSNIDTSKVNYGVTVLPTFKGQPSKPFVGVLSAGINAASPNKELAKEF
LENYLLTDEGLEAVNKDKPLGAVALKSYEEELAKDPRIAATMENAQKGEIMPNIPQMSAF
WYAVRTAVINAASGRQTVDEALKDAQTNSSSNNNNNNNNNNLGIEGRISEFASTDIELTQ
SPLSLPVSLGDQASISCTSSQSLLHSNGDTYLHWYLQKPGQSPKLLIYTLSNRFSGVPDRF
SGSGSGTDFTLKISRVEAADLGIYFCSQTTHVPYTFGGGTKLEIKRHHHHHHGAAEQKLIS
EEDLNGAA

In the case of using the supernatant, 344 g of ammonium sulfate was added to about 800 ml of the culture supernatant, and the mixture was stirred overnight at 4°C. Subsequently, an insoluble matter containing MBP-VH(BGP) or MBP-VL(BGP) was collected by centrifugation, and the pellet was suspended in 30 ml of TALON buffer. In the case of using the pellet of E. coli, the pellet was suspended in 30 ml of TALON buffer and then subjected to ultrasonication, followed by centrifugation, to collect a supernatant containing MBP-VH(BGP) or MBP-VL(BGP). The supernatant was dialyzed against the TALON buffer. Each protein collected in TALON buffer was applied to a column (16 mm-diameter × about 15 mm-height) filled with a TALON affinity resin (Clontech Laboratories, Inc., Mountain View, CA). Subsequently, TALON affinity resin, onto which the protein was adsorbed, was washed with the TALON buffer, and then a TALON elution buffer was added to elute MBP-VH(BGP) or MBP-VL(BGP). The purified protein was confirmed by SDS-PAGE. The buffer of the eluate was changed to HBS-N, and then glycerol was added thereto at a final concentration of 16%. The obtained solution was stored at -80°C.

(C) Preparation of MBP-VH(BGP) labeled with ALEXA FLUOR 555

100 μl of a solution containing the MBP-VH(BGP) obtained above (in HBS-N, about 1.2 mg/ml) was labeled with ALEXA FLUOR (registered trademark) 555 (manufactured by Molecular Probes). The labeling reaction was performed at pH 7.0.

(D) Preparation of MBP-VL(BGP) labeled with ALEXA FLUOR 647

430 μl of a solution containing the MBP-VL(BGP) obtained above (in HBS-N, about 150 μg/ml) was labeled with ALEXA FLUOR (registered trademark) 647 (manufactured by Molecular Probes). The labeling was performed at pH 7.0.

(2) Fabrication of a substrate provided with a carboxyl group

(A) Washing the substrate

A glass slide (manufactured by Matsunami Glass Ind., Ltd) was irradiated with UV, and immersed in acetone and ultrasonicated for 5 minutes. Subsequently, the glass slide was removed from acetone and washed with purified water.

(B) Providing an amino (APS) group

72 ml of ethanol and 8 ml of purified water were mixed in a 100 ml glass vial. The mixed solution was heated for about 15 minutes at 60°C in an incubator (manufactured by AS ONE Corporation). To the heated mixed solution of ethanol and purified water, 80 μl of APS (γ-aminopropyltriethoxysilane) (manufactured by Tokyo Chemical Industry Co., Ltd.) (0.1% v/v) was added and dissolved. Subsequently, the washed glass slide was immersed in this solution and allowed to react in the incubator (manufactured by AS ONE Corporation) for 15 minutes at 60°C, thereby obtaining a glass slide provided with the amino group. After the reaction, the glass slide was removed from the solution, and then washed by immersing the glass slide into a solution of ethanol and water at a ratio of 9:1 and then removing it. The washing was repeated three times. The glass slide after the washing was dried by blowing nitrogen gas thereon and baked for 1 hour at 120°C. After the baking, the glass slide was washed by sequentially immersing it in ethanol and purified water.

(C) Active esterification of CMD

To a 0.5% by mass solution of CMD (carboxymethyl dextran, manufactured by Meito Sangyo Co., Ltd., molecular weight 1,000,000) dissolved in ultrapure water, a mixed solution containing 0.4 M EDC (1-(3-Dimethylaminopropyl)-3-ethylcarbodiimide) and 0.1 M NHS (N-Hydroxysuccinimide) was added, being an amount calculated such that 2% of the carboxyl group in CMD is activated when the entire amount was reacted, and stirred for 5 minutes at room temperature.

(D) Preparation of a CMD film

The active esterified CMD solution was dropped onto the above prepared glass substrate having the amino group thereon. After 30 seconds, the solution was removed, whereby a thin film of active esterified carboxy methyl dextran was formed on the substrate having the amino group. The substrate was reacted for 1 hour at room temperature, and then washed once with 0.1 N NaOH and once with ultrapure water.

(3) Formation of a complex

MBP-VH(BGP) labeled with ALEXA FLUOR 555 and MBP-VL(BGP) labeled with ALEXA FLUOR 647, both of which were prepared above, and the C-terminal heptapeptide fragment of BGP (RRFYGPY: SEQ ID NO: 18) were mixed at a number ratio of 4:1:4 to form a solution containing 2 μM of MBP-VH(BGP) labeled with ALEXA FLUOR 555 in HEPES buffer. The mixed solution was left to stand for 3 hours at room temperature, thereby obtaining a complex sample solution.

(4) Immobilization of the complex

The CMD glass substrate prepared above was set in a surface prep unit of a surface plasmon resonance analyzer (trade name: BIACORE3000, manufactured by Biacore). Using the surface prep unit, 70 μl of a mixed aqueous solution of 0.2 mM EDC and 0.04 mMNHS was added to the glass substrate at a flow rate of 10 μl/min. Subsequently, 70 μl of a two-fold dilution of the above complex sample solution with an acetate buffer (pH 4.5) was injected onto the glass substrate, and then blocking treatment was performed with an ethanolamine solution to immobilize the complex.

(5) Washing of the antigen

After the immobilization of the complex, the substrate was subjected to 1-minute washing for five times by alternately using a glycine buffer (pH 1.5) and 10 mM NaOH to remove the antigen, thereby obtaining an immobilization substrate I of Example 7. The fluorescence intensity of the substrate after washing was measured using FLA-8000 (trade name, manufactured by FUJIFILM Corporation) and the intensity ratio between the emission at approximately 570 nm and the emission at approximately 675 nm was determined.

(6) Evaluation of the change in the amount of the fluorescence by the antigen

100 nM of the BGP heptapeptide (RRFYGPY) was injected onto the immobilization substrate I obtained above over 10 minutes, and then the substrate was washed with the HEPES buffer solution. Subsequently, the fluorescence intensity was measured by using FLA-8000 and the intensity ratio between the emission at approximately 570 nm and the emission at approximately 675 nm was determined. The intensity ratio between the emission at approximately 570 nm and the emission at approximately 675 nm obtained in this manner was divided by the intensity ratio between the emission at approximately 570 nm and the emission at approximately 675 nm obtained in section (5) above, thereby determining the change in the emission intensity ratio before and after the addition of the BGP heptapeptide (RRFYGPY).

Example 8

The immobilization substrate I used in Example 7 was subjected to 1-minute washing for five times by alternately using a glycine buffer (pH 1.5) and 10 mM NaOH to remove the antigen, thereby obtaining an immobilization substrate J of Example 8.

The fluorescence intensity of the immobilization substrate B was measured using FLA-8000 (trade name, manufactured by FUJIFILM Corporation) and the intensity ratio between the emission at approximately 570 nm and the emission at approximately 675 nm was determined.

Subsequently, 1 μM of BGP heptapeptide (RRFYGPY) was injected onto the immobilization substrate J over 10 minutes, and then the substrate was washed with the HEPES buffer solution. The fluorescence intensity was then measured by using FLA-8000 and the intensity ratio between the emission at approximately 570 nm and the emission at approximately 675 nm before and after the addition of the BGP heptapeptide (RRFYGPY) was determined

Comparative Example 3

An immobilization substrate K was prepared in the same manner as in Example 7, except that, in the immobilization of the complex, MBP-VH(BGP) labeled with ALEXA FLUOR 555 and MBP-VL(BGP) labeled with ALEXA FLUOR 647 were added to the substrate for an identical amount of time, in an alternating manner. The intensity ratio between the emission at approximately 570 nm and the emission at approximately 675 nm of the immobilization substrate K before and after the addition of the BGP heptapeptide (RRFYGPY) was determined in the same manner as in Example 7.

Determination of the change in the amount of the fluorescence before and after the addition of the BGP heptapeptide (RRFYGPY)

Based on the results obtained in Examples 7 and 8 and Comparative Example 3, the percentage of the change of the emission intensity ratio before and after the addition of the BGP heptapeptide (RRFYGPY) was determined based on the intensity ratio between the emission at approximately 570 nm and the emission at approximately 675 nm. The results are shown in Table 13.

Table 13

| | Immobilization substrate | The percentage of the change of the emission intensity ratio |
|---|---|---|
| Example 7 | I | 3% |
| Example 8 | J | 8% |
| Comparative Example 3 | K | 0% |

As is apparent from Table 13, a low-molecular-weight antigen can be detected by using the antibody-fragment immobilization substrate according to the present invention, which indicates a higher versatility of antigens. Since the change of the emission intensity in antibody-fragment immobilization substrates of Examples 7 and 8 depends on the concentration of the antigen even when a low-molecular-weight antigen is used, the concentration of the antigen can be quantified using the antibody-fragment immobilization substrate of the present invention. In the immobilization substrate K (Comparative Example 3), as is the case with lysozyme, the change was not detected even when a 10000-fold concentration (1 mM) of BGP heptapeptide (RRFYGPY) was used (data not shown).

Accordingly, the present invention provides an immobilization substrate that can precisely and simply detect a targeted molecule in a short time and that can be repeatedly used after washing. In addition, since the linker design is not required, the present invention also provides a method that can efficiently produce an immobilization substrate having greater versatility.

## Claims

1. An antibody-fragment-immobilizing substrate comprising a substrate and at least one set of antibody fragments, wherein antibody fragments of each set include at least two types of separate antibody fragments,
the at least two types of separate antibody fragments include at least one type of labeled antibody fragment having a labeled site labeled with a luminescent substance and at least one type of acceptor antibody fragment having an acceptor site for accepting emission from the labeled antibody fragment,
the at least one type of labeled antibody fragment and the at least one type of acceptor antibody fragment are capable of cooperatively recognizing one type of antigen and are independently immobilized on the substrate in a positional relationship that allows each of the antibody fragments in one set to bind to the same antigen, wherein upon binding the antigen the at least one type of labeled antibody fragment and the at least one type of acceptor antibody fragment approach one another, whereby a reaction based on luminescence occurs between said antibody fragments,
and
the antibody-fragment-immobilizing substrate is obtained by

contacting the separate antibody fragments with the antigen, and forming a complex whereby each of the antibody fragments binds to the antigen;
immobilizing the complex on a substrate via the antibody fragments in the complex; and
removing the antigen from the complex,
wherein the contacting includes mixing the antibody fragments and the antigen such that the ratio of the number of antigens to the valencies of molecules formed by a combination of antibody fragments is from 0.1:1 to 10:1.

**2.** The antibody-fragment-immobilizing substrate according to claim 1, wherein the at least two types of antibody fragments comprise a VH-region polypeptide and a VL-region polypeptide.

**3.** The antibody-fragment-immobilizing substrate according to claim 1 or claim 2, further comprising a polymer layer and wherein the at least two types of antibody fragments are immobilized on the polymer layer.

**4.** The antibody-fragment-immobilizing substrate according to claim 3, wherein the thickness of the polymer layer is from 1 nm to 0.5 mm.

**5.** The antibody-fragment-immobilizing substrate according to claim 3 or claim 4, wherein the polymer layer is bound to the substrate through a self-assembled monolayer.

**6.** The antibody-fragment-immobilizing substrate according claim 5, wherein the thickness of the self-assembled monolayer is from 0.2 nm to 10 μm.

**7.** The antibody-fragment-immobilizing substrate according to any one of claims 3 to 6, wherein the polymer is a polysaccharide.

**8.** The antibody-fragment-immobilizing substrate according to any one of claims 1 to 7, wherein the luminescent substance is at least one selected from a fluorescent dye or a fluorescent protein.

**9.** The antibody-fragment-immobilizing substrate according to any one of claims 1 to 8, wherein a fluorescence resonance energy transfer occurs between the labeled antibody fragment and the acceptor antibody fragment.

**10.** Use of an antibody-fragment-immobilizing substrate as defined in any one of claims 1 to 9 for a bioreactor or biosensor based on a binding reaction between the at least two types of antibody fragments and the antigen.

**11.** A method for producing an antibody-fragment-immobilizing substrate comprising:

contacting separate antibody fragments of at least two types that include at least one type of labeled antibody fragment having a labeled site labeled with a luminescent substance and at least one type of acceptor antibody fragment having an acceptor site for accepting emission from the labeled antibody fragment and that are capable of cooperatively recognizing one type of antigen, with the antigen, and forming a complex whereby each of the antibody fragments binds to the antigen;
immobilizing the complex on a substrate via the antibody fragments in the complex; and
removing the antigen from the complex to obtain the antibody-fragment-immobilizing substrate, wherein the antibody fragments are independently immobilized on the substrate in a positional relationship that allows each of the antibody fragments to bind to the same antigen
wherein the contacting includes mixing the antibody fragments and the antigen such that the ratio of the number of antigens to the valencies of molecules formed by a combination of antibody fragments is from 0.1:1 to 10:1.

**12.** The method for producing the antibody-fragment-immobilizing substrate according to claim 11, wherein the antibody fragments comprise a VH-region polypeptide and a VL-region polypeptide.

**13.** The method for producing the antibody-fragment-immobilizing substrate according to claim 11 or claim 12, wherein the removing is performed such that the avidity of the antibody fragments and the antigen in the complex is reduced.

**Patentansprüche**

**1.** Antikörperfragment-immobilisierendes Substrat, umfassend ein Substrat und mindestens einen Satz aus Antikörperfragmenten,
wobei Antikörperfragmente aus jedem Satz mindestens zwei Typen separater Antikörperfragmente umfassen,
wobei die mindestens zwei Typen separater Antikörperfragmente mindestens einen Typ von markiertem Antikörperfragment mit einer markierten Stelle, die mit einer lumineszierenden Substanz markiert ist, und mindestens einen Typ von Akzeptor-Antikörperfragment mit einer Akzeptor-Stelle zur Aufnahme von Emission von dem markierten Antikörperfragment umfassen,
wobei der mindestens eine Typ von markiertem Antikörperfragment und der mindestens eine Typ von Akzeptor-Antikörperfragment imstande sind, kooperativ einen Typ von Antigen zu erkennen, und unabhängig auf dem Substrat in einer räumlichen Anordnung immobilisiert sind, die erlaubt, dass jedes der Antikörperfragmente in einem Satz an das gleiche Antigen bindet, wobei der mindestens eine Typ von markiertem Antikörperfragment und der mindestens eine Typ von Akzeptor-Antikörperfragment nach Bindung an das Antigen sich annähern, wobei eine auf Lumineszenz basierende Reaktion zwischen den besagten Antikörperfragmenten stattfindet,
und
wobei das Antikörperfragment-immobilisierende Substrat erhalten wird durch
Inkontaktbringen der separaten Antikörperfragmente mit dem Antigen und Bilden eines Komplexes, wobei jedes der Antikörperfragmente an das Antigen bindet;
Immobilisieren des Komplexes auf einem Substrat durch die Antikörperfragmente in dem Komplex; und
Entfernen des Antigens aus dem Komplex,
wobei das Inkontaktbringen Mischen der Antikörperfragmente und des Antigens umfasst, so dass das Verhältnis der Anzahl von Antigenen zu den Valenzen von durch eine Kombination aus Antikörperfragmenten gebildeten Molekülen von 0,1:1 bis 10:1 beträgt.

2. Antikörperfragment-immobilisierendes Substrat gemäß Anspruch 1, wobei die mindestens zwei Typen von Antikörperfragmenten ein VH-Region-Polypeptid und ein VL-Region-Polypeptid umfassen.

3. Antikörperfragment-immobilisierendes Substrat gemäß Anspruch 1 oder Anspruch 2, ferner umfassend eine Polymerschicht und wobei die mindestens zwei Typen von Antikörperfragmenten auf der Polymerschicht immobilisiert sind.

4. Antikörperfragment-immobilisierendes Substrat gemäß Anspruch 3, wobei die Dicke der Polymerschicht von 1 nm bis 0,5 mm beträgt.

5. Antikörperfragment-immobilisierendes Substrat gemäß Anspruch 3 oder Anspruch 4, wobei die Polymerschicht an das Substrat durch eine selbstassemblierte Monoschicht gebunden ist.

6. Antikörperfragment-immobilisierendes Substrat gemäß Anspruch 5, wobei die Dicke der selbstassemblierten Monoschicht von 0,2 nm bis 10 $\mu$m beträgt.

7. Antikörperfragment-immobilisierendes Substrat gemäß einem der Ansprüche 3 bis 6, wobei das Polymer ein Polysaccharid ist.

8. Antikörperfragment-immobilisierendes Substrat gemäß einem der Ansprüche 1 bis 7, wobei die lumineszierende Substanz mindestens eine, ausgewählt aus einem Fluoreszenzfarbstoff oder einem Fluoreszenzprotein, ist.

9. Antikörperfragment-immobilisierendes Substrat gemäß einem der Ansprüche 1 bis 8, wobei ein Fluoreszenz-Resonanzenergietransfer zwischen dem markierten Antikörperfragment und dem Akzeptor-Antikörperfragment stattfindet.

10. Verwendung eines Antikörperfragment-immobilisierenden Substrats, definiert wie in einem der Ansprüche 1 bis 9, für einen Bioreaktor oder Biosensor, basierend auf einer Bindungsreaktion zwischen den mindestens zwei Typen von Antikörperfragmenten und dem Antigen.

11. Verfahren zum Herstellen eines Antikörperfragment-immobilisierenden Substrats, umfassend:

Inkontaktbringen separater Antikörperfragmente mindestens zweier Typen, die mindestens einen Typ von markiertem Antikörperfragment mit einer markierten Stelle, die mit einer lumineszierenden Substanz markiert ist, und mindestens einen Typ eines Akzeptor-Antikörperfragments mit einer Akzeptor-Stelle zur Aufnahme von Emission von dem markierten Antikörperfragment umfassen und die imstande sind, kooperativ einen Typ von Antigen zu erkennen, mit dem Antigen und Bilden eines Komplexes, wobei jedes der Antikörperfragmente an das Antigen bindet;
Immobilisieren des Komplexes auf einem Substrat durch die Antikörperfragmente in dem Komplex; und
Entfernen des Antigens aus dem Komplex, so dass das Antikörperfragment-immobilisierende Substrat erhalten wird, wobei die Antikörperfragmente unabhängig auf dem Substrat in einer Positionsbeziehung immobilisiert sind, die erlaubt, dass jedes der Antikörperfragmente an das gleiche Antigen bindet,
wobei das Inkontaktbringen Mischen der Antikörperfragmente und des Antigens umfasst, so dass das Verhältnis

der Anzahl von Antigenen zu den Valenzen von durch eine Kombination aus Antikörperfragmenten gebildeten Molekülen von 0,01:1 bis 10:1 beträgt.

**12.** Verfahren zum Herstellen des Antikörperfragment-immobilisierenden Substrats gemäß Anspruch 11, wobei die Antikörperfragmente ein VH-Region-Polypeptid und VL-Region-Polypeptid umfassen.

**13.** Verfahren zum Herstellen des Antikörperfragment-immobilisierenden Substrats gemäß Anspruch 11 oder Anspruch 12, wobei das Entfernen derart durchgeführt wird, dass die Avidität der Antikörperfragmente und des Antigens in dem Komplex reduziert wird.

## Revendications

**1.** Substrat d'immobilisation de fragments d'anticorps comprenant un substrat et au moins un ensemble de fragments d'anticorps,
où les fragments d'anticorps de chaque ensemble comprennent au moins deux types de fragments d'anticorps distincts,
les au moins deux types de fragments d'anticorps distincts comprennent au moins un type de fragment d'anticorps marqué possédant un site marqué qui est marqué avec une substance luminescente et au moins un type de fragment d'anticorps accepteur possédant un site accepteur pour accepter une émission à partir du fragment d'anticorps marqué,
le au moins un type de fragment d'anticorps marqué et le au moins un type de fragment d'anticorps accepteur sont capables de reconnaître de manière coopérative un type d'antigène et sont indépendamment immobilisés sur le substrat selon une relation positionnelle qui permet à chacun des fragments d'anticorps dans un ensemble de se lier au même antigène, où suite à la liaison de l'antigène, le au moins un type de fragment d'anticorps marqué et le au moins un type de fragment d'anticorps accepteur s'approchent l'un de l'autre, moyennant quoi une réaction basée sur la luminescence se produit entre lesdits fragments d'anticorps,
et
le substrat d'immobilisation de fragments d'anticorps est obtenu par
la mise en contact des fragments d'anticorps distincts avec l'antigène, et la formation d'un complexe moyennant quoi chacun des fragments d'anticorps se lie à l'antigène ;
l'immobilisation du complexe sur un substrat via les fragments d'anticorps dans le complexe ; et
l'élimination de l'antigène du complexe,
où la mise en contact comprend le mélange des fragments d'anticorps et de l'antigène de sorte que le rapport du nombre d'antigènes sur les valences des molécules formées par une combinaison de fragments d'anticorps soit compris entre 0,1:1 et 10:1.

**2.** Substrat d'immobilisation de fragments d'anticorps selon la revendication 1, dans lequel les au moins deux types de fragments d'anticorps comprennent un polypeptide de région VH et un polypeptide de région VL.

**3.** Substrat d'immobilisation de fragments d'anticorps selon la revendication 1 ou la revendication 2, comprenant en outre une couche polymère et où les au moins deux types de fragments d'anticorps sont immobilisés sur la couche polymère.

**4.** Substrat d'immobilisation de fragments d'anticorps selon la revendication 3, dans lequel l'épaisseur de la couche polymère est comprise entre 1 nm et 0,5 mm.

**5.** Substrat d'immobilisation de fragments d'anticorps selon la revendication 3 ou la revendication 4, dans lequel la couche polymère est liée au substrat par l'intermédiaire d'une monocouche auto-assemblée.

**6.** Substrat d'immobilisation de fragments d'anticorps selon la revendication 5, dans lequel l'épaisseur de la monocouche auto-assemblée est comprise entre 0,2 nm et 10 μm.

**7.** Substrat d'immobilisation de fragments d'anticorps selon l'une quelconque des revendications 3 à 6, dans lequel le polymère est un polysaccharide.

**8.** Substrat d'immobilisation de fragments d'anticorps selon l'une quelconque des revendications 1 à 7, dans lequel la substance luminescente est au moins une substance sélectionnée parmi un colorant fluorescent ou une protéine

fluorescente.

9. Substrat d'immobilisation de fragments d'anticorps selon l'une quelconque des revendications 1 à 8, dans lequel un transfert d'énergie par résonance de fluorescence se produit entre le fragment d'anticorps marqué et le fragment d'anticorps accepteur.

10. Utilisation d'un substrat d'immobilisation de fragments d'anticorps tel que défini dans l'une quelconque des revendications 1 à 9 pour un bioréacteur ou un biocapteur basé sur une réaction de liaison entre les au moins deux types de fragments d'anticorps et l'antigène.

11. Procédé de production d'un substrat d'immobilisation de fragments d'anticorps comprenant :

    la mise en contact de fragments d'anticorps distincts d'au moins deux types qui comprennent au moins un type de fragment d'anticorps marqué possédant un site marqué qui est marqué avec une substance luminescente et au moins un type de fragment d'anticorps accepteur possédant un site accepteur pour accepter une émission à partir du fragment d'anticorps marqué et qui sont capables de reconnaître de manière coopérative un type d'antigène, avec l'antigène, et la formation d'un complexe moyennant quoi chacun des fragments d'anticorps se lie à l'antigène ;
    l'immobilisation du complexe sur un substrat via les fragments d'anticorps dans le complexe ; et
    l'élimination de l'antigène du complexe afin d'obtenir le substrat d'immobilisation de fragments d'anticorps, où les fragments d'anticorps sont indépendamment immobilisés sur le substrat selon une relation positionnelle qui permet à chacun des fragments d'anticorps de se lier au même antigène,
    où la mise en contact comprend le mélange des fragments d'anticorps et de l'antigène de sorte que le rapport du nombre d'antigènes sur les valences des molécules formées par une combinaison de fragments d'anticorps soit compris entre 0,1:1 et 10:1.

12. Procédé de production du substrat d'immobilisation de fragments d'anticorps selon la revendication 11, dans lequel les fragments d'anticorps comprennent un polypeptide de région VH et un polypeptide de région VL.

13. Procédé de production du substrat d'immobilisation de fragments d'anticorps selon la revendication 11 ou la revendication 12, dans lequel l'élimination est réalisée de sorte que l'avidité des fragments d'anticorps et de l'antigène dans le complexe soit réduite.

FIG.1A

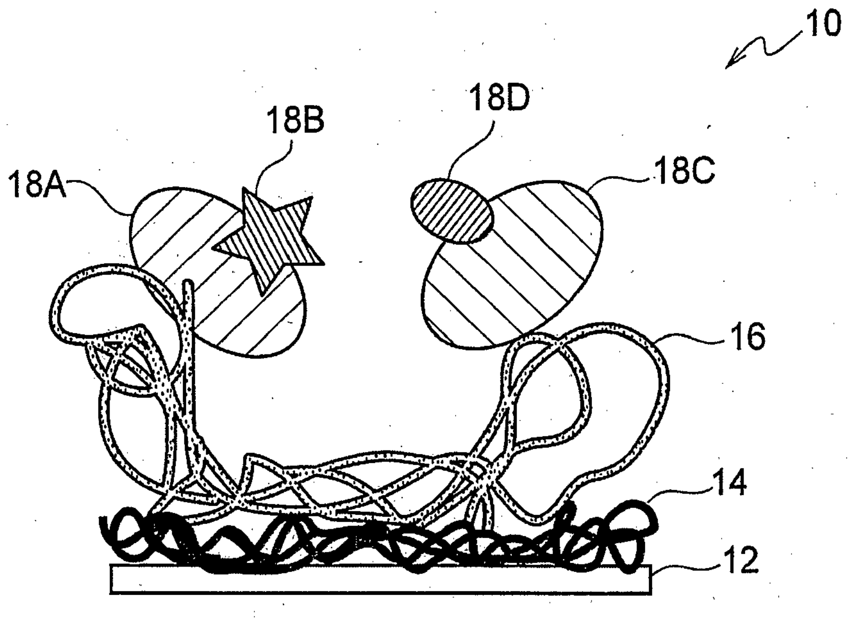


FIG.1B

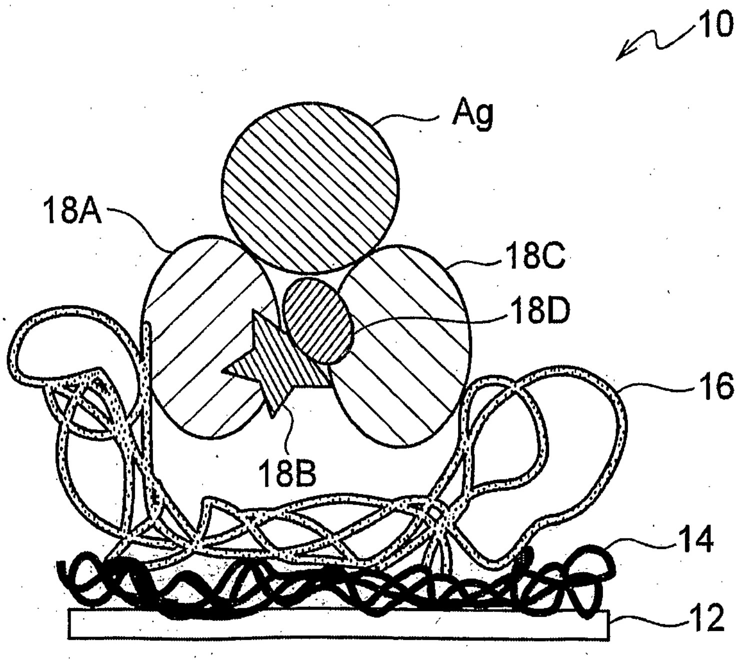

FIG.2

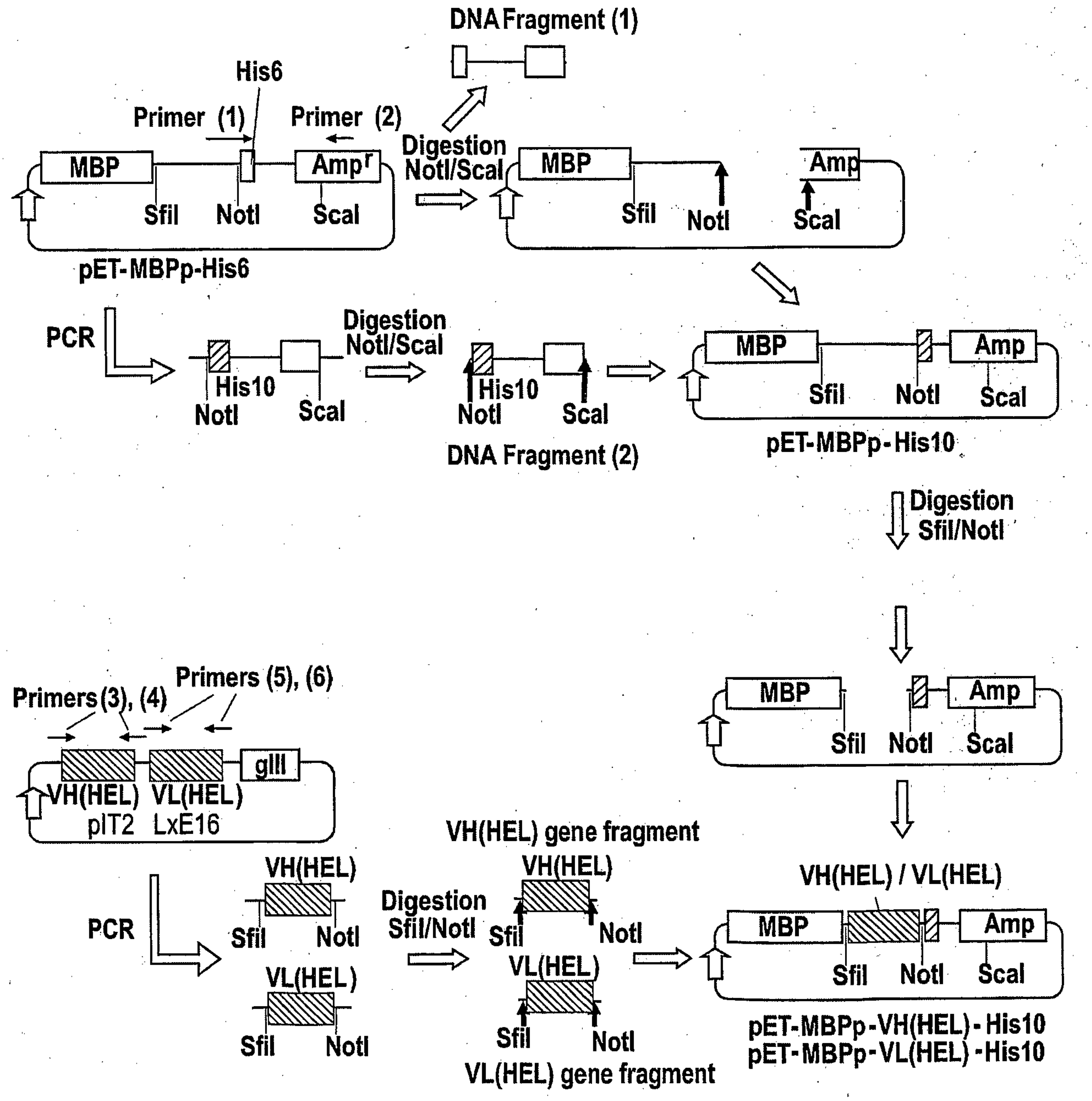
DNA Fragment (1)
His6
Primer (1)
Primer (2)
MBP
Amp^r
SfiI
NotI
ScaI
pET-MBPp-His6
Digestion NotI/ScaI
MBP
Amp
SfiI
NotI
ScaI
PCR
His10
NotI
ScaI
Digestion NotI/ScaI
His10
NotI
ScaI
DNA Fragment (2)
MBP
Amp
SfiI
NotI
ScaI
pET-MBPp-His10
Digestion SfiI/NotI
MBP
Amp
SfiI
NotI
ScaI
Primers (3), (4)
Primers (5), (6)
gIII
VH(HEL)
VL(HEL)
pIT2 LxE16
PCR
VH(HEL)
SfiI
NotI
VL(HEL)
SfiI
NotI
Digestion SfiI/NotI
VH(HEL) gene fragment
VH(HEL)
SfiI
NotI
VL(HEL)
SfiI
NotI
VL(HEL) gene fragment
VH(HEL) / VL(HEL)
MBP
Amp
SfiI
NotI
ScaI
pET-MBPp-VH(HEL)-His10
pET-MBPp-VL(HEL)-His10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006138656 A
- JP 10078436 A
- JP 2007040834 A
- JP 2008083042 A
- WO 2010047419 A
- JP 59053836 A
- JP 59071048 A
- JP 2006058071 A
- JP 2006090781 A
- JP 2007523754 B
- JP 2001519525 B
- JP 2001089482 A
- JP 4018398 Y
- JP 4018399 A
- JP 2008309839 A
- JP 2009117081 A


### Non-patent literature cited in the description


- **UEDA et al.** Open sandwich ELISA: A novel immunoassay based on the interchain interaction of antibody variable region. *Nature Biotechnology,* 1996, vol. 14, 1714-1718
- *Chemical Review,* 2005, vol. 105, 1103-1169
- **YUJI HARAZAKI.** Progress in Coating Technology (Coating Gijutsu no Shinpo). Sogo Gijyutsu Center, 1988
- Coating Technology (Coating Gijutsu). Technical Information Institute Co., Ltd, 1999
- Aqueous Coating Technology (Suisei Coating no Gijutsu). CMC, 2001
- Evolving Organic Thin Film. Sumibe Techno Research Co., Ltd, 2004
- **AKIRA IWAMORI.** Polymer Surface Processing Technology (Polymer Hyomen Kako Gaku). Gihodo Shuppan Co., Ltd, 2005
- *BIOPHYSICS (Seibutsu Butsuri),* 2002, vol. 42 (1), 28-31
- Bioreactor Technique. CMC Publishing Co., Ltd, 1988
- Biochip and Biosensor. Kyoritsu Shuppan Co., Ltd, 2006
- Laboratory of Protein Engineering, Department of Chemistry and Biotechnology, Graduate School of Engineering. University of Tokyo
- **LIM et al.** *Anal. Chem.,* 2007, vol. 79, 6193

SEQUENCE LISTING

<110> FUJIFILM Corporation
The University of Tokyo

<120> IMMOBILIZATION SUBSTRATE AND METHOD FOR PRODUCING THE SAME

<130> FS-F05022-00

<150> JP2008-309839
<151> 2008-12-04

<150> JP2009-117081
<151> 2009-5-13

<160> 18

<170> PatentIn version 3.4

<210> 1
<211> 59
<212> DNA
<213> Artificial

<220>
<223> primer (1)

<400> 1
aaaaaaagcg gccgcggagc atcatcacca tcaccaccac caccaccact gagatccgg 59

<210> 2
<211> 18
<212> DNA
<213> Artificial

<220>
<223> primer (2)

<400> 2
ccaatgctta atcagtga 18

<210> 3
<211> 49
<212> DNA
<213> Artificial

<220>
<223> primer (3)

<400> 3
ctttctatgc ggcccagccg gccatggccg akgtsvagct tcaggagtc 49

<210> 4
<211> 34
<212> DNA
<213> Artificial.

<220>
<223> primer (4)

<400> 4
aaaaaagcgg ccgcgctcga gacggtgacc gtgg 34

<210> 5
<211> 45
<212> DNA
<213> Artificial

<220>
<223> primer (5)

<400> 5
aaaaaaggcc cagccggcca tggcgtcgac ggatattttg atgac 45

<210> 6
<211> 36
<212> DNA
<213> Artificial

<220>
<223> primer (6)

<400> 6
tttctcgtgc ggccgcacgt tttatttcca actttg 36

<210> 7
<211> 5217
<212> DNA
<213> Artificial

<220>
<223> pET-MBP-His6

<400> 7

ctagaaataa ttttgtttaa ctttaagaag gagatataca tatgaaaata aaaacaggtg 60
cacgcatcct cgcattatcc gcattaacga cgatgatgtt ttccgcctcg gctctcgcca 120
aaatcgaaga aggtaaactg gtaatctgga ttaacggcga taaaggctat aacggtctcg 180
ctgaagtcgg taagaaattc gagaaagata ccggaattaa agtcaccgtt gagcatccgg 240
ataaactgga agagaaattc ccacaggttg cggcaactgg cgatggccct gacattatct 300
tctgggcaca cgaccgcttt ggtggctacg ctcaatctgg cctgttggct gaaatcaccc 360
cggacaaagc gttccaggac aagctgtatc cgtttacctg ggatgccgta cgttacaacg 420
gcaagctgat tgcttacccg atcgctgttg aagcgttatc gctgatttat aacaaagatc 480
tgctgccgaa cccgccaaaa acctgggaag agatcccggc gctggataaa gaactgaaag 540
cgaaaggtaa gagcgcgctg atgttcaacc tgcaagaacc gtacttcacc tggccgctga 600
ttgctgctga cgggggttat gcgttcaagt atgaaaacgg caagtacgac attaaagacg 660
tgggcgtgga taacgctggc gcgaaagcgg gtctgacctt cctggttgac ctgattaaaa 720
acaaacacat gaatgcagac accgattact ccatcgcaga agctgccttt aataaaggcg 780
aaacagcgat gaccatcaac ggcccgtggg catggtccaa catcgacacc agcaaagtga 840
attatggtgt aacggtactg ccgaccttca agggtcaacc atccaaaccg ttcgttggcg 900
tgctgagcgc aggtattaac gccgccagtc cgaacaaaga gctggcaaaa gagttcctcg 960
aaaactatct gctgactgat gaaggtctgg aagcggttaa taaagacaaa ccgctgggtg 1020
ccgtagcgct gaagtcttac gaggaagagt tggcgaaaga tccacgtatt gccgccacca 1080
tggaaaacgc ccagaaaggt gaaatcatgc cgaacatccc gcagatgtcc gctttctggt 1140
atgccgtgcg tactgcggtg atcaacgccg ccagcggtcg tcagactgtc gatgaagccc 1200
tgaaagacgc gcagactaat tcgagctcga acaacaacaa caataacaat aacaacaacc 1260
tcgggccggg tgcggcacac tacgtagaat tcgcggccca gccggccatg gccgactaca 1320
aagatattgt gctgacacag tctcctgctt ccttagctgt atctctgggg cagagggcca 1380
ccatctcatg cagggccagc caaagtgtca gtacatctac ctatagttat ttacactggt 1440
accaacagag accaggacag ccacccaaac tcatcaagta tgtatccaac ctagaatctg 1500
gggtccctgc caggttcagt ggcagtgggt ctgggacaga cttcaccctc aacatccatc 1560

ctgtggagga ggaggatact gcaacatatt actgtcagca cagttgggag attcctccga 1620
cgttcggtgg aggcaccaag ctggaaataa aacgtgcggc cgcggtcgag caccaccacc 1680
accaccactg agatccggct gctaacaaag cccgaaagga agctgagttg gctgctgcca 1740
ccgctgagca ataactagca taaccccttg gggcctctaa acgggtcttg aggggttttt 1800
tgctgaaagg aggaactata tccggattgg cgaatgggac gcgccctgta gcggcgcatt 1860
aagcgcggcg ggtgtggtgg ttacgcgcag cgtgaccgct acacttgcca gcgccctagc 1920
gcccgctcct ttcgctttct tcccttcctt tctcgccacg ttcgccggct ttccccgtca 1980
agctctaaat cgggggctcc ctttagggtt ccgatttagt gctttacggc acctcgaccc 2040
caaaaaactt gattagggtg atggttcacg tagtgggcca tcgccctgat agacggtttt 2100
tcgccctttg acgttggagt ccacgttctt taatagtgga ctcttgttcc aaactggaac 2160
aacactcaac cctatctcgg tctattcttt tgatttataa gggattttgc cgatttcggc 2220
ctattggtta aaaaatgagc tgatttaaca aaaatttaac gcgaatttta acaaaatatt 2280
aacgtttaca atttcaggtg gcacttttcg gggaaatgtg cgcggaaccc ctatttgttt 2340
atttttctaa atacattcaa atatgtatcc gctcatgaga caataaccct gataaatgct 2400
tcaataatat tgaaaaagga agagtatgag tattcaacat ttccgtgtcg cccttattcc 2460
cttttttgcg gcattttgcc ttcctgtttt tgctcaccca gaaacgctgg tgaaagtaaa 2520
agatgctgaa gatcagttgg gtgcacgagt gggttacatc gaactggatc tcaacagcgg 2580
taagatcctt gagagttttc gccccgaaga acgttttcca atgatgagca cttttaaagt 2640
tctgctatgt ggcgcggtat tatcccgtat tgacgccggg caagagcaac tcggtcgccg 2700
catacactat tctcagaatg acttggttga gtactcacca gtcacagaaa agcatcttac 2760
ggatggcatg acagtaagag aattatgcag tgctgccata accatgagtg ataacactgc 2820
ggccaactta cttctgacaa cgatcggagg accgaaggag ctaaccgctt ttttgcacaa 2880
catgggggat catgtaactc gccttgatcg ttgggaaccg gagctgaatg aagccatacc 2940
aaacgacgag cgtgacacca cgatgcctgc agcaatggca acaacgttgc gcaaactatt 3000
aactggcgaa ctacttactc tagcttcccg gcaacaatta atagactgga tggaggcgga 3060
taaagttgca ggaccacttc tgcgctcggc ccttccggct ggctggttta ttgctgataa 3120
atctggagcc ggtgagcgtg ggtctcgcgg tatcattgca gcactggggc cagatggtaa 3180
gccctcccgt atcgtagtta tctacacgac ggggagtcag gcaactatgg atgaacgaaa 3240
tagacagatc gctgagatag gtgcctcact gattaagcat tggtaactgt cagaccaagt 3300
ttactcatat atactttaga ttgatttaaa acttcatttt taatttaaaa ggatctaggt 3360
gaagatcctt tttgataatc tcatgaccaa aatcccttaa cgtgagtttt cgttccactg 3420
agcgtcagac cccgtagaaa agatcaaagg atcttcttga gatccttttt ttctgcgcgt 3480
aatctgctgc ttgcaaacaa aaaaaccacc gctaccagcg gtggtttgtt tgccggatca 3540
agagctacca actctttttc cgaaggtaac tggcttcagc agagcgcaga taccaaatac 3600
tgtccttcta gtgtagccgt agttaggcca ccacttcaag aactctgtag caccgcctac 3660
atacctcgct ctgctaatcc tgttaccagt ggctgctgcc agtggcgata agtcgtgtct 3720
taccgggttg gactcaagac gatagttacc ggataaggcg cagcggtcgg gctgaacggg 3780
gggttcgtgc acacagccca gcttggagcg aacgacctac accgaactga gatacctaca 3840

gcgtgagcta tgagaaagcg ccacgcttcc cgaagggaga aaggcggaca ggtatccggt 3900
aagcggcagg gtcggaacag gagagcgcac gagggagctt ccagggggaa acgcctggta 3960
tctttatagt cctgtcgggt ttcgccacct ctgacttgag cgtcgatttt tgtgatgctc 4020
gtcagggggg cggagcctat ggaaaaacgc cagcaacgcg gcctttttac ggttcctggc 4080
cttttgctgg ccttttgctc acatgttctt tcctgcgtta tcccctgatt ctgtggataa 4140
ccgtattacc gcctttgagt gagctgatac cgctcgccgc agccgaacga ccgagcgcag 4200
cgagtcagtg agcgaggaag cggaagagcg cctgatgcgg tattttctcc ttacgcatct 4260
gtgcggtatt tcacaccgca tatatggtgc actctcagta caatctgctc tgatgccgca 4320
tagttaagcc agtatacact ccgctatcgc tacgtgactg ggtcatggct gcgccccgac 4380
acccgccaac acccgctgac gcgccctgac gggcttgtct gctcccggca tccgcttaca 4440
gacaagctgt gaccgtctcc gggagctgca tgtgtcagag gttttcaccg tcatcaccga 4500
aacgcgcgag gcagctgcgg taaagctcat cagcgtggtc gtgaagcgat tcacagatgt 4560
ctgcctgttc atccgcgtcc agctcgttga gtttctccag aagcgttaat gtctggcttc 4620
tgataaagcg ggccatgtta agggcggttt tttcctgttt ggtcactgat gcctccgtgt 4680
aagggggatt tctgttcatg ggggtaatga taccgatgaa acgagagagg atgctcacga 4740
tacgggttac tgatgatgaa catgcccggt tactggaacg ttgtgagggt aaacaactgg 4800
cggtatggat gcggcgggac cagagaaaaa tcactcaggg tcaatgccag cgcttcgtta 4860
atacagatgt aggtgttcca cagggtagcc agcagcatcc tgcgatgcag atccggaaca 4920
taatggtgca gggcgctgac ttccgcgttt ccagacttta cgaaacacgg aaaccgaaga 4980
ccattcatgt tgttgctcag gtcgcagacg ttttgcagca gcagtcgctt cacgttcgct 5040
cgcgtatcgg tgattcattc tgctaaccag taaggcaacc ccgccagcct agccgggtcc 5100
tcaacgacag gagcacgatc atgcgcaccc gtggccagga cccaacgctg cccgagatct 5160
cgatcccgcg aaattaatac gactcactat agggagacca caacggtttc cctctag 5217

<210> 8
<211> 840
<212> DNA
<213> Artificial

<220>
<223> pIT2-LxE16

<220>
<221 CDS
<222> (29).. (838)

<400> 8

```
caaattctat ttcaaggaga cagtcata atg aaa tac cta ttg cct acg gca      52
                               Met Lys Tyr Leu Leu Pro Thr Ala
                               1               5

gcc gct gga ttg tta tta ctc gcg gcc cag ccg gcc atg gcc gag gtg     100
Ala Ala Gly Leu Leu Leu Leu Ala Ala Gln Pro Ala Met Ala Glu Val
    10                  15                  20

cag ctt cag gag tca gga cct agc ctc gtg aaa cct tct cag act ctg     148
Gln Leu Gln Glu Ser Gly Pro Ser Leu Val Lys Pro Ser Gln Thr Leu
25                  30                  35                  40

tcc ctc acc tgt tct gtc act ggc gac tcc atc acc agg ggt tac tgg     196
Ser Leu Thr Cys Ser Val Thr Gly Asp Ser Ile Thr Arg Gly Tyr Trp
                45                  50                  55

agc tgg atc cgg aaa tcc cca gga aat aaa ctt gag tac atg ggg tac     244
Ser Trp Ile Arg Lys Ser Pro Gly Asn Lys Leu Glu Tyr Met Gly Tyr
            60                  65                  70

ata agc tac agt ggt agc act ttc tac aat cca tct ctc aaa agt cga     292
Ile Ser Tyr Ser Gly Ser Thr Phe Tyr Asn Pro Ser Leu Lys Ser Arg
        75                  80                  85

atc tcc atc act cga gac aca ttc aag aac cag ctc tac ctg cag ttg     340
Ile Ser Ile Thr Arg Asp Thr Phe Lys Asn Gln Leu Tyr Leu Gln Leu
    90                  95                  100

aat tct gtg act act gag gac aca gcc aca tat tat tgt gca gag tac     388
Asn Ser Val Thr Thr Glu Asp Thr Ala Thr Tyr Tyr Cys Ala Glu Tyr
105                 110                 115                 120

gac ggg act tac tgg ggc caa ggg acc acg gtc acc gtc tcg agc ggt     436
Asp Gly Thr Tyr Trp Gly Gln Gly Thr Thr Val Thr Val Ser Ser Gly
                125                 130                 135

gga ggc ggt tca ggc gga ggt ggc agc ggc ggt ggc ggg tcg acg gat     484
Gly Gly Gly Ser Gly Gly Gly Gly Ser Gly Gly Gly Gly Ser Thr Asp
            140                 145                 150

att ttg atg acc cag act cca gcc acc ctg tct gtg act cca gga gat     532
Ile Leu Met Thr Gln Thr Pro Ala Thr Leu Ser Val Thr Pro Gly Asp
        155                 160                 165

agc gtc agt ctt tcc tgc agg gcc agc caa agt att ggc aac aac cta     580
Ser Val Ser Leu Ser Cys Arg Ala Ser Gln Ser Ile Gly Asn Asn Leu
    170                 175                 180

cac tgg ttt caa caa aaa tca cat gag tct cca agg ctt ctc atc aag     628
His Trp Phe Gln Gln Lys Ser His Glu Ser Pro Arg Leu Leu Ile Lys
185                 190                 195                 200

tat gct tcc cag tcc atc tct ggg atc ccc tcc agg ttc agt ggc agt     676
Tyr Ala Ser Gln Ser Ile Ser Gly Ile Pro Ser Arg Phe Ser Gly Ser
                205                 210                 215

gga tca ggg aca gat ttc act ctc agt atc aac act gtg gag act gaa     724
Gly Ser Gly Thr Asp Phe Thr Leu Ser Ile Asn Thr Val Glu Thr Glu
            220                 225                 230

gat ttt gga atg tat ttc tgt caa cag agt aac agc tgg ccg tac acg     772
Asp Phe Gly Met Tyr Phe Cys Gln Gln Ser Asn Ser Trp Pro Tyr Thr
        235                 240                 245

ttc gga ggg ggg aca aag ttg gaa ata aaa cgt gcg gcc gca cat cat     820
Phe Gly Gly Gly Thr Lys Leu Glu Ile Lys Arg Ala Ala Ala His His
    250                 255                 260

cat cac cat cac ggg gcc gc                                          840
His His His His Gly Ala
265                 270
```

<210> 9
<211> 270
<212> PRT
<213> Artificial

<220>
<223> Synthetic Construct

<400> 9

Met Lys Tyr Leu Leu Pro Thr Ala Ala Ala Gly Leu Leu Leu Leu Ala
1 5 10 15

Ala Gln Pro Ala Met Ala Glu Val Gln Leu Gln Glu Ser Gly Pro Ser
20 25 30

Leu Val Lys Pro Ser Gln Thr Leu Ser Leu Thr Cys Ser Val Thr Gly
35 40 45

Asp Ser Ile Thr Arg Gly Tyr Trp Ser Trp Ile Arg Lys Ser Pro Gly
50 55 60

Asn Lys Leu Glu Tyr Met Gly Tyr Ile Ser Tyr Ser Gly Ser Thr Phe
65 70 75 80

Tyr Asn Pro Ser Leu Lys Ser Arg Ile Ser Ile Thr Arg Asp Thr Phe
85 90 95

Lys Asn Gln Leu Tyr Leu Gln Leu Asn Ser Val Thr Thr Glu Asp Thr
100 105 110

Ala Thr Tyr Tyr Cys Ala Glu Tyr Asp Gly Thr Tyr Trp Gly Gln Gly
115 120 125

Thr Thr Val Thr Val Ser Ser Gly Gly Gly Gly Ser Gly Gly Gly Gly
130 135 140

Ser Gly Gly Gly Gly Ser Thr Asp Ile Leu Met Thr Gln Thr Pro Ala
145 150 155 160

Thr Leu Ser Val Thr Pro Gly Asp Ser Val Ser Leu Ser Cys Arg Ala
165 170 175

Ser Gln Ser Ile Gly Asn Asn Leu His Trp Phe Gln Gln Lys Ser His
180 185 190

Glu Ser Pro Arg Leu Leu Ile Lys Tyr Ala Ser Gln Ser Ile Ser Gly
195 200 205

Ile Pro Ser Arg Phe Ser Gly Ser Gly Ser Gly Thr Asp Phe Thr Leu
210 215 220

Ser Ile Asn Thr Val Glu Thr Glu Asp Phe Gly Met Tyr Phe Cys Gln
225 230 235 240

Gln Ser Asn Ser Trp Pro Tyr Thr Phe Gly Gly Gly Thr Lys Leu Glu
245 250 255

Ile Lys Arg Ala Ala Ala His His His His His His Gly Ala
260 265 270

<210> 10
<211> 341
<212> DNA
<213> Artificial

<220>
<223> VH(HEL)

<400> 10

atggccgagg tgcagcttca ggagtcagga cctagcctcg tgaaaccttc tcagactctg 60
tccctcacct gttctgtcac tggcgactcc atcaccaggg gttactggag ctggatccgg 120
aaatccccag gaaataaact tgagtacatg ggtacataa gctacagtgg tagcactttc 180
tacaatccat ctctcaaaag tcgaatctcc atcactcgag acacattcaa gaaccagctc 240

tacctgcagt tgaattctgt gactactgag gacacagcca catattattg tgcagagtac 300
gacgggactt actggggcca agggaccacg gtcaccgtct c 341

<210> 11
<211> 330
<212> DNA
<213> Artificial

<220>
<223> VL(HEL)

<400> 11

tcgacggata ttttgatgac ccagactcca gccaccctgt ctgtgactcc aggagatagc 60
gtcagtcttt cctgcagggc cagccaaagt attggcaaca acctacactg gtttcaacaa 120
aaatcacatg agtctccaag gcttctcatc aagtatgctt cccagtccat ctctgggatc 180
ccctccaggt tcagtggcag tggatcaggg acagatttca ctctcagtat caacactgtg 240
gagactgaag attttggaat gtatttctgt caacagagta acagctggcc gtacacgttc 300
ggaggggga caaagttgga aataaaacgt 330

<210> 12
<211> 551
<212> PRT
<213> Artificial

<220>
<223> MBP-VH(HEL)-His10

<400> 12

```
Met Lys Ile Lys Thr Gly Ala Arg Ile Leu Ala Leu Ser Ala Leu Thr
1               5                   10                  15

Thr Met Met Phe Ser Ala Ser Ala Leu Ala Lys Ile Glu Glu Gly Lys
            20                  25                  30

Leu Val Ile Trp Ile Asn Gly Asp Lys Gly Tyr Asn Gly Leu Ala Glu
        35                  40                  45

Val Gly Lys Lys Phe Glu Lys Asp Thr Gly Ile Lys Val Thr Val Glu
    50                  55                  60

His Pro Asp Lys Leu Glu Glu Lys Phe Pro Gln Val Ala Ala Thr Gly
65                  70                  75                  80

Asp Gly Pro Asp Ile Ile Phe Trp Ala His Asp Arg Phe Gly Gly Tyr
                85                  90                  95

Ala Gln Ser Gly Leu Leu Ala Glu Ile Thr Pro Asp Lys Ala Phe Gln
            100                 105                 110

Asp Lys Leu Tyr Pro Phe Thr Trp Asp Ala Val Arg Tyr Asn Gly Lys
        115                 120                 125

Leu Ile Ala Tyr Pro Ile Ala Val Glu Ala Leu Ser Leu Ile Tyr Asn
    130                 135                 140

Lys Asp Leu Leu Pro Asn Pro Pro Lys Thr Trp Glu Glu Ile Pro Ala
145                 150                 155                 160
```

Leu Asp Lys Glu Leu Lys Ala Lys Gly Lys Ser Ala Leu Met Phe Asn
165 170 175

Leu Gln Glu Pro Tyr Phe Thr Trp Pro Leu Ile Ala Ala Asp Gly Gly
180 185 190

Tyr Ala Phe Lys Tyr Glu Asn Gly Lys Tyr Asp Ile Lys Asp Val Gly
195 200 205

Val Asp Asn Ala Gly Ala Lys Ala Gly Leu Thr Phe Leu Val Asp Leu
210 215 220

Ile Lys Asn Lys His Met Asn Ala Asp Thr Asp Tyr Ser Ile Ala Glu
225 230 235 240

Ala Ala Phe Asn Lys Gly Glu Thr Ala Met Thr Ile Asn Gly Pro Trp
245 250 255

Ala Trp Ser Asn Ile Asp Thr Ser Lys Val Asn Tyr Gly Val Thr Val
260 265 270

Leu Pro Thr Phe Lys Gly Gln Pro Ser Lys Pro Phe Val Gly Val Leu
275 280 285

Ser Ala Gly Ile Asn Ala Ala Ser Pro Asn Lys Glu Leu Ala Lys Glu
290 295 300

Phe Leu Glu Asn Tyr Leu Leu Thr Asp Glu Gly Leu Glu Ala Val Asn
305 310 315 320

Lys Asp Lys Pro Leu Gly Ala Val Ala Leu Lys Ser Tyr Glu Glu Glu
325 330 335

Leu Ala Lys Asp Pro Arg Ile Ala Ala Thr Met Glu Asn Ala Gln Lys
340 345 350

Gly Glu Ile Met Pro Asn Ile Pro Gln Met Ser Ala Phe Trp Tyr Ala
355 360 365

Val Arg Thr Ala Val Ile Asn Ala Ala Ser Gly Arg Gln Thr Val Asp
370 375 380

Glu Ala Leu Lys Asp Ala Gln Thr Asn Ser Ser Ser Asn Asn Asn Asn
385 390 395 400

Asn Asn Asn Asn Asn Asn Leu Gly Pro Gly Ala Ala His Tyr Val Glu
405 410 415

Phe Ala Ala Gln Pro Ala Met Ala Asp Val Glu Leu Gln Glu Ser Gly
420 425 430

Pro Ser Leu Val Lys Pro Ser Gln Thr Leu Ser Leu Thr Cys Ser Val
435 440 445

Thr Gly Asp Ser Ile Thr Arg Gly Tyr Trp Ser Trp Ile Arg Lys Phe
450 455 460

Pro Gly Asn Lys Leu Glu Tyr Met Gly Tyr Ile Ser Tyr Ser Gly Ser
465 470 475 480

Thr Phe Tyr Asn Pro Ser Leu Lys Ser Arg Ile Ser Ile Thr Arg Asp
485 490 495

Thr Phe Lys Asn Gln Leu Tyr Leu Gln Leu Asn Ser Val Thr Thr Glu
500 505 510

Asp Thr Ala Thr Tyr Tyr Cys Ala Glu Tyr Asp Gly Thr Tyr Trp Gly
515 520 525

Gln Gly Thr Thr Val Thr Val Ser Ser Ala Ala Ala Glu His His His
530 535 540

His His His His His His His
545 550

<210> 13
<211> 548
<212> PRT
<213> Artificial

<220>
<223> MBP-VL(HEL)-His10

<400> 13

Met Lys Ile Lys Thr Gly Ala Arg Ile Leu Ala Leu Ser Ala Leu Thr
1 5 10 15

Thr Met Met Phe Ser Ala Ser Ala Leu Ala Lys Ile Glu Glu Gly Lys
20 25 30

Leu Val Ile Trp Ile Asn Gly Asp Lys Gly Tyr Asn Gly Leu Ala Glu
35 40 45

Val Gly Lys Lys Phe Glu Lys Asp Thr Gly Ile Lys Val Thr Val Glu
50 55 60

His Pro Asp Lys Leu Glu Glu Lys Phe Pro Gln Val Ala Ala Thr Gly
65 70 75 80

Asp Gly Pro Asp Ile Ile Phe Trp Ala His Asp Arg Phe Gly Gly Tyr
85 90 95

Ala Gln Ser Gly Leu Leu Ala Glu Ile Thr Pro Asp Lys Ala Phe Gln
100 105 110

Asp Lys Leu Tyr Pro Phe Thr Trp Asp Ala Val Arg Tyr Asn Gly Lys
115 120 125

Leu Ile Ala Tyr Pro Ile Ala Val Glu Ala Leu Ser Leu Ile Tyr Asn
130 135 140

Lys Asp Leu Leu Pro Asn Pro Pro Lys Thr Trp Glu Glu Ile Pro Ala
145 150 155 160

Leu Asp Lys Glu Leu Lys Ala Lys Gly Lys Ser Ala Leu Met Phe Asn
165 170 175

Leu Gln Glu Pro Tyr Phe Thr Trp Pro Leu Ile Ala Ala Asp Gly Gly
180 185 190

Tyr Ala Phe Lys Tyr Glu Asn Gly Lys Tyr Asp Ile Lys Asp Val Gly
195 200 205

Val Asp Asn Ala Gly Ala Lys Ala Gly Leu Thr Phe Leu Val Asp Leu
210 215 220

Ile Lys Asn Lys His Met Asn Ala Asp Thr Asp Tyr Ser Ile Ala Glu
225 230 235 240

Ala Ala Phe Asn Lys Gly Glu Thr Ala Met Thr Ile Asn Gly Pro Trp
245 250 255

Ala Trp Ser Asn Ile Asp Thr Ser Lys Val Asn Tyr Gly Val Thr Val
260 265 270

Leu Pro Thr Phe Lys Gly Gln Pro Ser Lys Pro Phe Val Gly Val Leu
275 280 285

Ser Ala Gly Ile Asn Ala Ala Ser Pro Asn Lys Glu Leu Ala Lys Glu
290 295 300

Phe Leu Glu Asn Tyr Leu Leu Thr Asp Glu Gly Leu Glu Ala Val Asn
305 310 315 320

Lys Asp Lys Pro Leu Gly Ala Val Ala Leu Lys Ser Tyr Glu Glu Glu
325 330 335

Leu Ala Lys Asp Pro Arg Ile Ala Ala Thr Met Glu Asn Ala Gln Lys
340 345 350

Gly Glu Ile Met Pro Asn Ile Pro Gln Met Ser Ala Phe Trp Tyr Ala
355 360 365

Val Arg Thr Ala Val Ile Asn Ala Ala Ser Gly Arg Gln Thr Val Asp
370 375 380

Glu Ala Leu Lys Asp Ala Gln Thr Asn Ser Ser Ser Asn Asn Asn Asn
385 390 395 400

Asn Asn Asn Asn Asn Asn Leu Gly Pro Gly Ala Ala His Tyr Val Glu
405 410 415

Phe Ala Ala Gln Pro Ala Met Ala Ser Thr Asp Ile Leu Met Thr Gln
420 425 430

Thr Pro Ala Thr Leu Ser Val Thr Pro Gly Asp Ser Val Ser Leu Ser
435 440 445

Cys Arg Ala Ser Gln Ser Ile Gly Asn Asn Leu His Trp Phe Gln Gln
450 455 460

```
Lys Ser His Glu Ser Pro Arg Leu Leu Ile Lys Tyr Ala Ser Gln Ser
465                 470                 475                 480

Ile Ser Gly Ile Pro Ser Arg Phe Ser Gly Ser Gly Ser Gly Thr Asp
                485                 490                 495

Phe Thr Leu Ser Ile Asn Thr Val Glu Thr Glu Asp Phe Gly Met Tyr
            500                 505                 510

Phe Cys Gln Gln Ser Asn Ser Trp Pro Tyr Thr Phe Gly Gly Gly Thr
        515                 520                 525

Lys Leu Glu Ile Lys Arg Ala Ala Ala Glu His His His His His His
    530                 535                 540

His His His His
545
```

<210> 14
<211> 348
<212> DNA
<213> Artificial

<220>
<223> VH(BGP)

<220>
<221> CDS
<222> (1).. (348)

<400> 14

```
gag gta cag ctg gag gag tct ggg gct gag ttt gtg aag gct ggg gct      48
Glu Val Gln Leu Glu Glu Ser Gly Ala Glu Phe Val Lys Ala Gly Ala
1               5                   10                  15

tca gtg aag ctg tcc tgc aag act tct ggc tac acc ttc aac aac tac      96
Ser Val Lys Leu Ser Cys Lys Thr Ser Gly Tyr Thr Phe Asn Asn Tyr
            20                  25                  30

tgg att cac tgg gtc aaa cag agc cca gga caa ggc ctt gaa tgg atc      144
Trp Ile His Trp Val Lys Gln Ser Pro Gly Gln Gly Leu Glu Trp Ile
        35                  40                  45

gga gaa att gat ccc tct gat ggt tat tct aac tac aat caa aaa ttc      192
Gly Glu Ile Asp Pro Ser Asp Gly Tyr Ser Asn Tyr Asn Gln Lys Phe
    50                  55                  60

aag ggc aag gcc aca ttg act gtg gac aag tcc tcc agc act gcc tac      240
Lys Gly Lys Ala Thr Leu Thr Val Asp Lys Ser Ser Ser Thr Ala Tyr
65                  70                  75                  80

atg cac ctc aac agt ctg act tct gag gac tct gcg gtc tat tat tgt      288
Met His Leu Asn Ser Leu Thr Ser Glu Asp Ser Ala Val Tyr Tyr Cys
                85                  90                  95

aca agc agc act tcg gta gga ggt tcc tgg ggc caa ggg acc acg gtc      336
Thr Ser Ser Thr Ser Val Gly Gly Ser Trp Gly Gln Gly Thr Thr Val
            100                 105                 110

acc gtc tcg agc                                                      348
Thr Val Ser Ser
        115
```

<210> 15
<211> 116
<212> PRT
<213> Artificial

<220>
<223> Synthetic Construct

<400> 15

Glu Val Gln Leu Glu Glu Ser Gly Ala Glu Phe Val Lys Ala Gly Ala
1 5 10 15

Ser Val Lys Leu Ser Cys Lys Thr Ser Gly Tyr Thr Phe Asn Asn Tyr
20 25 30

Trp Ile His Trp Val Lys Gln Ser Pro Gly Gln Gly Leu Glu Trp Ile
35 40 45

Gly Glu Ile Asp Pro Ser Asp Gly Tyr Ser Asn Tyr Asn Gln Lys Phe
50 55 60

Lys Gly Lys Ala Thr Leu Thr Val Asp Lys Ser Ser Ser Thr Ala Tyr
65 70 75 80

Met His Leu Asn Ser Leu Thr Ser Glu Asp Ser Ala Val Tyr Tyr Cys
85 90 95

Thr Ser Ser Thr Ser Val Gly Gly Ser Trp Gly Gln Gly Thr Thr Val
100 105 110

Thr Val Ser Ser
115

<210> 16
<211> 544
<212> PRT
<213> Artificial

<220>
<223> MBP-VH(BGP)

<400> 16

```
Met Lys Ile Lys Thr Gly Ala Arg Ile Leu Ala Leu Ser Ala Leu Thr
1               5                   10                  15

Thr Met Met Phe Ser Ala Ser Ala Leu Ala Lys Ile Glu Glu Gly Lys
            20                  25                  30

Leu Val Ile Trp Ile Asn Gly Asp Lys Gly Tyr Asn Gly Leu Ala Glu
        35                  40                  45

Val Gly Lys Lys Phe Glu Lys Asp Thr Gly Ile Lys Val Thr Val Glu
    50                  55                  60

His Pro Asp Lys Leu Glu Glu Lys Phe Pro Gln Val Ala Ala Thr Gly
65                  70                  75                  80

Asp Gly Pro Asp Ile Ile Phe Trp Ala His Asp Arg Phe Gly Gly Tyr
                85                  90                  95

Ala Gln Ser Gly Leu Leu Ala Glu Ile Thr Pro Asp Lys Ala Phe Gln
            100                 105                 110

Asp Lys Leu Tyr Pro Phe Thr Trp Asp Ala Val Arg Tyr Asn Gly Lys
```

115 120 125

Leu Ile Ala Tyr Pro Ile Ala Val Glu Ala Leu Ser Leu Ile Tyr Asn
130 135 140

Lys Asp Leu Leu Pro Asn Pro Pro Lys Thr Trp Glu Glu Ile Pro Ala
145 150 155 160

Leu Asp Lys Glu Leu Lys Ala Lys Gly Lys Ser Ala Leu Met Phe Asn
165 170 175

Leu Gln Glu Pro Tyr Phe Thr Trp Pro Leu Ile Ala Ala Asp Gly Gly
180 185 190

Tyr Ala Phe Lys Tyr Glu Asn Gly Lys Tyr Asp Ile Lys Asp Val Gly
195 200 205

Val Asp Asn Ala Gly Ala Lys Ala Gly Leu Thr Phe Leu Val Asp Leu
210 215 220

Ile Lys Asn Lys His Met Asn Ala Asp Thr Asp Tyr Ser Ile Ala Glu
225 230 235 240

Ala Ala Phe Asn Lys Gly Glu Thr Ala Met Thr Ile Asn Gly Pro Trp
245 250 255

Ala Trp Ser Asn Ile Asp Thr Ser Lys Val Asn Tyr Gly Val Thr Val
260 265 270

Leu Pro Thr Phe Lys Gly Gln Pro Ser Lys Pro Phe Val Gly Val Leu
275 280 285

Ser Ala Gly Ile Asn Ala Ala Ser Pro Asn Lys Glu Leu Ala Lys Glu
290 295 300

Phe Leu Glu Asn Tyr Leu Leu Thr Asp Glu Gly Leu Glu Ala Val Asn
305 310 315 320

Lys Asp Lys Pro Leu Gly Ala Val Ala Leu Lys Ser Tyr Glu Glu Glu
325 330 335

Leu Ala Lys Asp Pro Arg Ile Ala Ala Thr Met Glu Asn Ala Gln Lys
340 345 350

Gly Glu Ile Met Pro Asn Ile Pro Gln Met Ser Ala Phe Trp Tyr Ala
355 360 365

Val Arg Thr Ala Val Ile Asn Ala Ala Ser Gly Arg Gln Thr Val Asp
370 375 380

Glu Ala Leu Lys Asp Ala Gln Thr Asn Ser Ser Ser Asn Asn Asn Asn
385 390 395 400

Asn Asn Asn Asn Asn Asn Leu Gly Pro Gly Ala Ala His Tyr Val Glu
405 410 415

Phe Ala Ala Gln Pro Ala Glu Val Gln Leu Glu Glu Ser Gly Ala Glu

420 425 430

Phe Val Lys Ala Gly Ala Ser Val Lys Leu Ser Cys Lys Thr Ser Gly
435 440 445

Tyr Thr Phe Asn Ser Tyr Trp Ile His Trp Ile Lys Gln Ser Pro Gly
450 455 460

Gln Gly Leu Glu Trp Ile Gly Glu Ile Asp Pro Ser Asp Gly Tyr Thr
465 470 475 480

Asn Tyr Asn Gln Lys Phe Lys Gly Lys Ala Thr Leu Thr Val Asp Lys
485 490 495

Ser Ser Ser Thr Ala Tyr Met Arg Leu Asn Ser Leu Thr Ser Glu Asp
500 505 510

Ser Ala Val Tyr Tyr Cys Thr Ser Ser Thr Ser Val Gly Gly Ser Trp
515 520 525

Gly Gln Gly Thr Thr Val Thr Val Ser Ser His His His His His His
530 535 540

<210> 17
<211> 555
<212> PRT
<213> Artificial

<220>
<223> MBP-VL(BGP)

<400> 17

Met Lys Ile Lys Thr Gly Ala Arg Ile Leu Ala Leu Ser Ala Leu Thr
1 5 10 15

Thr Met Met Phe Ser Ala Ser Ala Leu Ala Lys Ile Glu Glu Gly Lys
20 25 30

Leu Val Ile Trp Ile Asn Gly Asp Lys Gly Tyr Asn Gly Leu Ala Glu
35 40 45

Val Gly Lys Lys Phe Glu Lys Asp Thr Gly Ile Lys Val Thr Val Glu
50 55 60

His Pro Asp Lys Leu Glu Glu Lys Phe Pro Gln Val Ala Ala Thr Gly
65 70 75 80

Asp Gly Pro Asp Ile Ile Phe Trp Ala His Asp Arg Phe Gly Gly Tyr
85 90 95

Ala Gln Ser Gly Leu Leu Ala Glu Ile Thr Pro Asp Lys Ala Phe Gln
100 105 110

Asp Lys Leu Tyr Pro Phe Thr Trp Asp Ala Val Arg Tyr Asn Gly Lys
115 120 125

Leu Ile Ala Tyr Pro Ile Ala Val Glu Ala Leu Ser Leu Ile Tyr Asn
130 135 140

Lys Asp Leu Leu Pro Asn Pro Pro Lys Thr Trp Glu Glu Ile Pro Ala
145 150 155 160

Leu Asp Lys Glu Leu Lys Ala Lys Gly Lys Ser Ala Leu Met Phe Asn
165 170 175

Leu Gln Glu Pro Tyr Phe Thr Trp Pro Leu Ile Ala Ala Asp Gly Gly
180 185 190

Tyr Ala Phe Lys Tyr Glu Asn Gly Lys Tyr Asp Ile Lys Asp Val Gly
195 200 205

Val Asp Asn Ala Gly Ala Lys Ala Gly Leu Thr Phe Leu Val Asp Leu
210 215 220

Ile Lys Asn Lys His Met Asn Ala Asp Thr Asp Tyr Ser Ile Ala Glu
225 230 235 240

Ala Ala Phe Asn Lys Gly Glu Thr Ala Met Thr Ile Asn Gly Pro Trp
245 250 255

Ala Trp Ser Asn Ile Asp Thr Ser Lys Val Asn Tyr Gly Val Thr Val
260 265 270

Leu Pro Thr Phe Lys Gly Gln Pro Ser Lys Pro Phe Val Gly Val Leu
275 280 285

Ser Ala Gly Ile Asn Ala Ala Ser Pro Asn Lys Glu Leu Ala Lys Glu
290 295 300

Phe Leu Glu Asn Tyr Leu Leu Thr Asp Glu Gly Leu Glu Ala Val Asn
305 310 315 320

Lys Asp Lys Pro Leu Gly Ala Val Ala Leu Lys Ser Tyr Glu Glu Glu
325 330 335

Leu Ala Lys Asp Pro Arg Ile Ala Ala Thr Met Glu Asn Ala Gln Lys
340 345 350

Gly Glu Ile Met Pro Asn Ile Pro Gln Met Ser Ala Phe Trp Tyr Ala
355 360 365

Val Arg Thr Ala Val Ile Asn Ala Ala Ser Gly Arg Gln Thr Val Asp
370 375 380

Glu Ala Leu Lys Asp Ala Gln Thr Asn Ser Ser Ser Asn Asn Asn Asn
385 390 395 400

Asn Asn Asn Asn Asn Asn Leu Gly Ile Glu Gly Arg Ile Ser Glu Phe
405 410 415

Ala Ser Thr Asp Ile Glu Leu Thr Gln Ser Pro Leu Ser Leu Pro Val
420 425 430

Ser Leu Gly Asp Gln Ala Ser Ile Ser Cys Thr Ser Ser Gln Ser Leu
435 440 445

Leu His Ser Asn Gly Asp Thr Tyr Leu His Trp Tyr Leu Gln Lys Pro
450 455 460

Gly Gln Ser Pro Lys Leu Leu Ile Tyr Thr Leu Ser Asn Arg Phe Ser
465 470 475 480

Gly Val Pro Asp Arg Phe Ser Gly Ser Gly Ser Gly Thr Asp Phe Thr
485 490 495

Leu Lys Ile Ser Arg Val Glu Ala Ala Asp Leu Gly Ile Tyr Phe Cys
500 505 510

Ser Gln Thr Thr His Val Pro Tyr Thr Phe Gly Gly Gly Thr Lys Leu
515 520 525

Glu Ile Lys Arg His His His His His His Gly Ala Ala Glu Gln Lys
530 535 540

Leu Ile Ser Glu Glu Asp Leu Asn Gly Ala Ala
545 550 555

<210> 18
<211> 7
<212> PRT
<213> Artificial

<220>
<223> BGP flagment

<400> 18

Arg Arg Phe Tyr Gly Pro Tyr
1 5